# EUROPEAN PATENT SPECIFICATION

(11) **EP 3 415 215 B1**
(45) Date of publication and mention of the grant of the patent: **18.12.2019**
(21) Application number: 18177829.1
(22) Date of filing: 14.06.2018
(51) Int. Cl.: B01D 39/20

(54) **FILTER, FILTER ASSEMBLY AND FILTER SYSTEM THEREOF**
FILTER, FILTERANORDNUNG UND FILTERSYSTEM DAFÜR
FILTRE, ENSEMBLE DE FILTRE ET SON SYSTÈME DE FILTRE

(30) Priority: 16.06.2017 TW 106120153; 03.04.2018 TW 107111936
(43) Date of publication of application: 19.12.2018
(73) Proprietor: AUO Crystal Corporation, Taichung City 421 (TW)
(72) Inventor: LEE, Tung-Feng, Taichung City 421 (TW); HSU, Meng-Kwei, Taichung City 406 (TW)
(74) Representative: Agasse, Stéphane

(56) References cited:
- EP-A1- 3 475 458
- WO-A1-2009/085553
- JP-A- 2016 155 118
- US-A1- 2010 098 877

## Description

The disclosure relates to a water composition, particularly to a water composition containing silicic acid and hydrogen gas. The disclosure also relates to a filter, a filter assembly and a water purification system for producing the water composition. The disclosure further relates to a method for making the water composition, use of the water composition, a water composition set, and a water composition-generating portable product.

Hydrogen-dissolved drinking water is advantageous to human beings for it can neutralize reactive oxygen species or free radicals present in the body. Therefore, research topics related to hydrogen-dissolved drinking water has become popular in recent years.

Currently, most commonly sold hydrogen-dissolved drinking water is produced by directly dissolving high-purity hydrogen in water, or reacting magnesium powder or a magnesium tablet with pure water to generate hydrogen gas. However, the former method has problems such as difficulty in obtaining high-purity hydrogen, difficulty in dissolving hydrogen in water, and safety concerns over use of high-purity hydrogen. With regard to the latter method, magnesium hydroxide that is produced by reacting magnesium with water may not be simultaneously taken with some drugs used for treating cardiovascular diseases. Moreover, if the content of magnesium hydroxide is too high, it is likely to lead to acute drug poisoning, acute renal failure, hypermagnesemia or other adverse health conditions.

Based on the above, safely producing hydrogen-dissolved drinking water which is beneficial to living organisms (e.g., human beings) remains a problem to be solved by those skilled in the art.

WO 2009/085553 A1 discloses a filtration media comprising an adsorptive media (carrier) and plasma-treated polymeric binder particles, wherein the surfaces of the plasma-treated polymeric binder particles comprise an oxide, silicon, or both. The filtration media is used for water treatment.

US Patent Application Publication No. 2017/0043305 A1 discloses an apparatus and a method of manufacturing hydrogen-containing drinking water, in which hydrogen generated by the electrolysis of water was dissolved in water by applying pressure. However, power consumption in this method would be large, and the use of pressurized hydrogen in water also has safety concerns.

Based on the above, safely producing hydrogen-dissolved drinking water which is beneficial to living organisms (e.g., human beings) remains a problem to be solved by those skilled in the art.

Therefore, an object of the disclosure is to provide a filter, a filter assembly, and a water purification system that can alleviate at least one of the drawbacks of the prior art.

According to one aspect of the disclosure, a filter for producing a water composition containing silicic acid and hydrogen gas includes a carrier and a silicon material supported on the carrier.

According to another aspect of the disclosure, a filter assembly includes a housing and the aforesaid filter. The housing defines a receiving space and includes an inlet and an outlet. The inlet and the outlet are in fluid communication with the receiving space. The filter is received in the receiving space of the housing.

According to yet another aspect of the disclosure, a water purification system includes at least one the aforesaid filter assembly.

Other features and advantages of the present disclosure will become apparent in the following detailed description of the embodiment with reference to the accompanying drawings, of which:
FIG. 1 is a schematic view showing a first embodiment of a water composition according to the present disclosure which is contained in a first container;
FIG. 2 is a schematic view illustrating step (a) and step (b) of a method for making the first embodiment of the water composition;
FIG. 3 illustrates step (a1), step (a2) and step (a3) of the step (a) of the method for making the first embodiment of the water composition;
FIG. 4 illustrates step (a4), step (a4') and step (a5) of the step (a) of the method for making the first embodiment of the water composition;
FIG. 5 is a schematic view illustrating step (c) of the method for making the first embodiment of the water composition;
FIG. 6 is a schematic view of a first embodiment of a filter according to the present disclosure;
FIG. 7 is a perspective view showing a method for making the first embodiment of the filter;
FIG. 8 is a fragmentary schematic view of a second embodiment of the filter;
FIG. 9 is a schematic view showing a third embodiment of the filter and a method for making the same;
FIG. 10 is a fragmentary schematic view showing a first embodiment of a filter assembly according to the present disclosure;
FIG. 11 is a fragmentary schematic view showing a second embodiment of the filter assembly;
FIG. 12 is a fragmentary schematic view showing a third embodiment of the filter assembly illustrating a state before usage;
FIG. 13 is a fragmentary schematic view showing the third embodiment of the filter assembly illustrating a state after usage;
FIG. 14 is a fragmentary schematic view showing a fourth embodiment of the filter assembly;
FIG. 15 is a fragmentary schematic view showing a fifth embodiment of the filter assembly;
FIG. 16 is a fragmentary schematic view showing a sixth embodiment of the filter assembly;
FIG. 17 is a fragmentary schematic view showing a first embodiment of a water purification system according to the present disclosure;
FIG. 18 is a fragmentary schematic view showing a second embodiment of the water composition produced by the first embodiment of the water purification system;
FIG. 19 is a fragmentary schematic view showing a second embodiment of the water purification system;
FIG. 20 is a fragmentary enlarged view illustrating a detailed structure of a second filter assembly used in the second embodiment of the water purification system shown in FIG. 19,;
FIG. 21 is a fragmentary schematic view showing a third embodiment of the water purification system;
FIG. 22 is a graph of oxidation-reduction potential (ORP) versus time, demonstrating stability of the water composition (produced using the water purification system shown in FIG. 17) under different filling ratio; and
FIG. 23 is photographs showing different color change between test sample A (containing apple juice and the water composition of the present disclosure) and test sample B (containing apple juice and untreated drinking water).

Before the disclosure is described in greater detail, it should be noted that where considered appropriate, reference numerals or terminal portions of reference numerals have been repeated among the figures to indicate corresponding or analogous elements, which may optionally have similar characteristics.

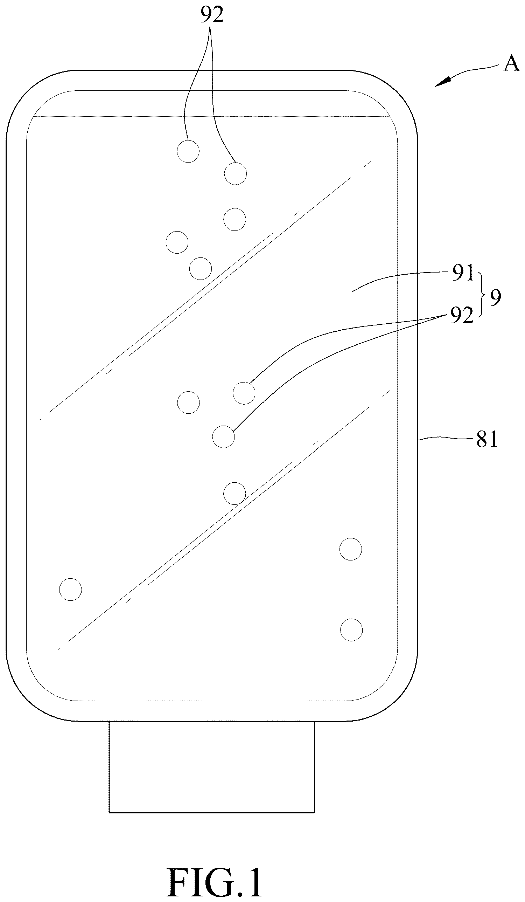

Referring to Figure 1, a first embodiment of a water composition 9 according to the present disclosure is shown to be sealed in a first container 81 that is inverted upside down. The water composition 9 includes water 91, silicic acid dissolved in the water 91, and hydrogen gas 92 dissolved in the water 91. In this embodiment, the water 91 substantially fills the first container 81, and the concentration of the silicic acid (i.e., the concentration of dissolved silica) present in the water composition 9 is not greater than the saturation concentration of the silicic acid. The oxidation-reduction potential (hereinafter abbreviated as ORP) of the water composition 9 is lower than -400 mV. The water composition 9 and the first container 81 constitute a water composition set (A).

According to this disclosure, the water 91 can be defined as water that is utilizable by living organisms (e.g., animals, plants, and other organisms) . It should be noted herein that the atomic weight of the hydrogen gas 92 is extremely light, so that the hydrogen gas 92 dissolved in the water 91 can easily float upwards. Accordingly, the purpose of inverting the first container 81 upside down is that, the hydrogen gas 92 that drifts upward is positioned at the bottom of the first container 81 and is dissolved in the water 91. As such, when the first container 81 returns to its normal position from the upside down position, and is opened to release its sealed state for drinking, most of the hydrogen gas 92 will still remain at the bottom of the first container 81 and will not be quickly moved out of the first container 81 due to the release of the sealed state.

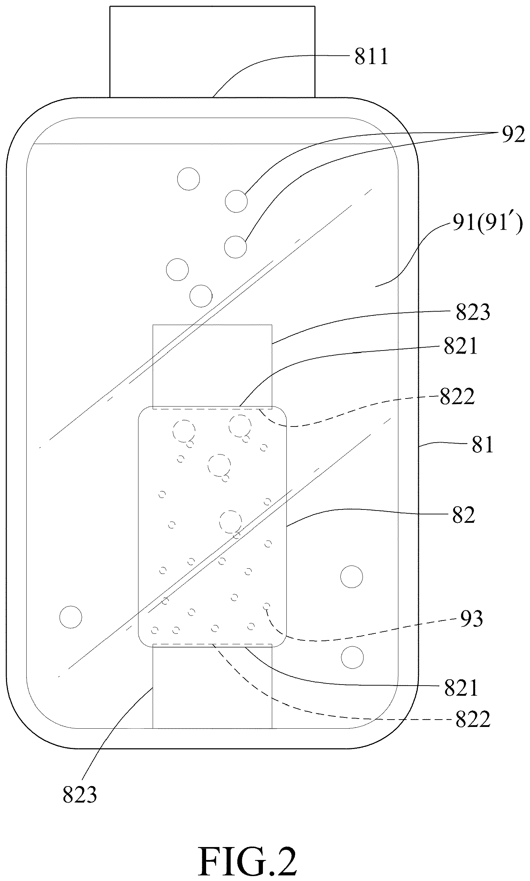

In certain embodiments, the silicic acid and the hydrogen gas 92 are generated by mixing a to-be-treated water 91' (hereinafter referred to as to-be-treated water 91') with a predetermined amount of silicon material 93 (see FIG. 2) . That is, the silicon material 93 can be used to make the above-mentioned water composition 9 by mixing with the to-be-treated water 91' . The silicon material 93 is nano silicon. Examples of the silicon material 93 include non-porous silicon, porous silicon, granulated silicon, silicon nanowire and combinations thereof. In an exemplary embodiment, the content of the predetermined amount of the silicon material 93 in the water 91 ranges between 40 mg/L to 200 mg/L. The silicon material 93 is nano silicon. In certain embodiment, the silicon material 93 has an average diameter ranging between 50 nm and 300 nm. In another exemplary embodiment, the average diameter of the silicon material 93 ranges between 100 nm and 250 nm. The nano silicon is highly reactive in nature, which facilitates rapid reaction with water molecules to generate hydrogen gas and silicic acid. It should be further explained herein that, the smaller the average diameter of the nano silicon is, the greater the activity thereof is, and the faster the reaction rate between the nano silicon and the water 91 is. In order to prevent the nano silicon from being consumed rather quickly in view of the fact that its average diameter is very small and hence its reaction with water is vigorous, in certain embodiments, the nano silicon is covered with a silicon oxide film.

The nano silicon may be discarded nano silicon powder yielded from squaring and slicing Si ingot. The porous silicon may be a silicon wafer, silicon powder, etc. that has been subjected to acid etching. The granulated silicon may be produced by spray granulation of silicon powder. The silicon nanowire may be obtained by etching a silicon material using an etchant.

In an exemplary embodiment, the concentration of the silicic acid in the water composition 9 ranges between 11 mg/L and the saturation concentration of the silicic acid. In another exemplary embodiment, the concentration of the silicic acid in the water composition 9 is between 20 mg/L and its saturation concentration, whereas the ORP of the water composition 9 is lower than -500 mV. In a further exemplary embodiment, the concentration of the silicic acid in the water composition 9 is between 40 mg/L and its saturation concentration; the ORP of the water composition 9 is below -650 mV. Parenthetically, the concentration of the silicic acid should not exceed its saturation concentration in the water composition 9 so as to avoid supersaturation of the silicic acid that may cause the silicic acid to polymerize or precipitate into insoluble silica gel.

In pharmaceutical industry or even in healthcare-related fields, silicon and silicic acid are known to be essential for the formation of bones and connective tissues, and are conducive in treating osteoporosis, nail fracture, hair loss, increased wrinkle and other health issues. Therefore, when the first embodiment of the water composition 9 of this disclosure is used as drinking water, the silicic acid in the water composition 9 will be beneficial for the formation of human connective tissues.

Referring to FIGS. 2 to 5, a method for making the first embodiment of the water composition 9 according to the present disclosure includes the following steps (a), (b) and (c).

As shown in FIG. 2, the step (a) includes filling water to be treated 91' into the first container 81 from an opening 811 of the first container 81, and mixing the predetermined amount of the silicon material 93 with the to-be-treated water 91'. The step (b) includes sealing the opening 811 of the first container 81 that is filled with a mixture of the to-be-treated water 91' and the predetermined amount of the silicon material 93. In this embodiment, the to-be-treated water 91' substantially fills the first container 81, and the to-be-treated water 91' and the silicon material 93 are reacted with each other to generate the silicic acid and the hydrogen gas 92 which are dissolved in the treated water 91, thereby obtaining the water composition 9. The average diameter of the silicon material 93, the predetermined amount of the silicon material 93 in the water 91, the concentration of the silicic acid dissolved in the water composition 9 and the ORP of the water composition 9 have been described in detail in the foregoing, and will not be further described hereinafter.

To be specific, step (a) includes sub-steps (a1), (a2), (a3), (a4) and (a5).

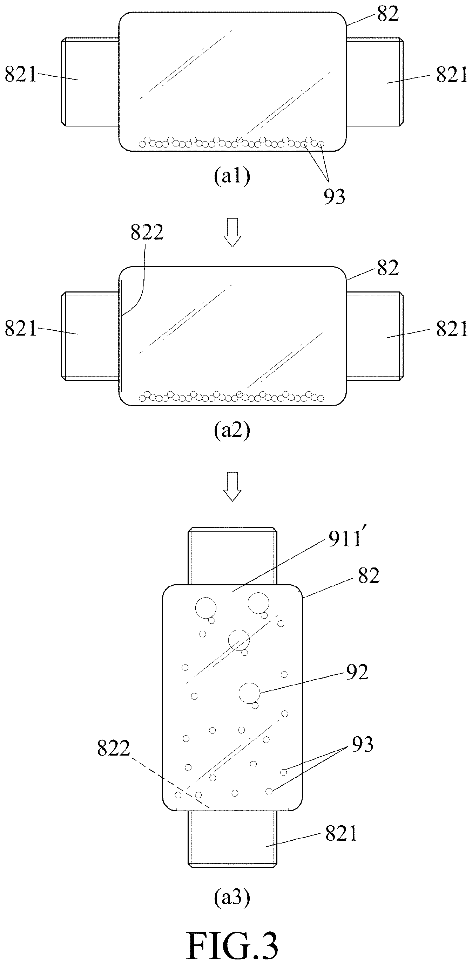

As shown in FIGS. 2 and 3, the sub-step (a1) is adding the predetermined amount of the silicon material 93 into a second container 82 having a smaller capacity than the first container 81. The second container 82 has two opposite openings 821.

The sub-step (a2) is covering one of the openings 821 of the second container 82 with a hydrophilic membrane filter 822.

The sub-step (a3) is, after the sub-step (a2), filling a portion 911' of the to-be-treated water 91' in the second container 82 so that the portion 911' of the to-be-treated water 91' is reacted with the predetermined amount of the silicon material 93 so as to generate the silicic acid and the hydrogen gas 92.

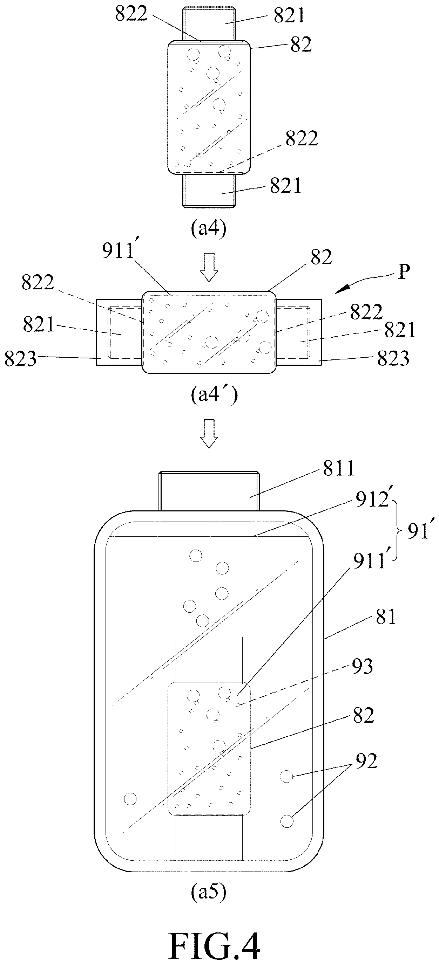

As shown in FIG. 4, the sub-step (a4) is covering the other of the openings 821 of the second container 82 using an additional hydrophilic membrane filter 822.

The sub-step (a5) is, after the sub-step (a4), disposing the second container 82 and a remaining portion 912' of the to-be-treated water 91' into the first container 81 from the opening 811 of the first container 81 so that the predetermined amount of the silicon material 93 and the remaining portion 912' of the to-be-treated water 91' continues to be reacted with each other so as to further generate the silicic acid and the hydrogen gas 92. In this embodiment, the portion 911' of the to-be-treated water 91' is first filled in the second container 82. However, in other embodiments, the portion 911' of the to-be-treated water 91' may not be filled in the second container 82, and the predetermined amount of the silicon material 93 in the second container 82 is directly reacted with the portion 912' of the to-be treated water 91' in the first container 81.

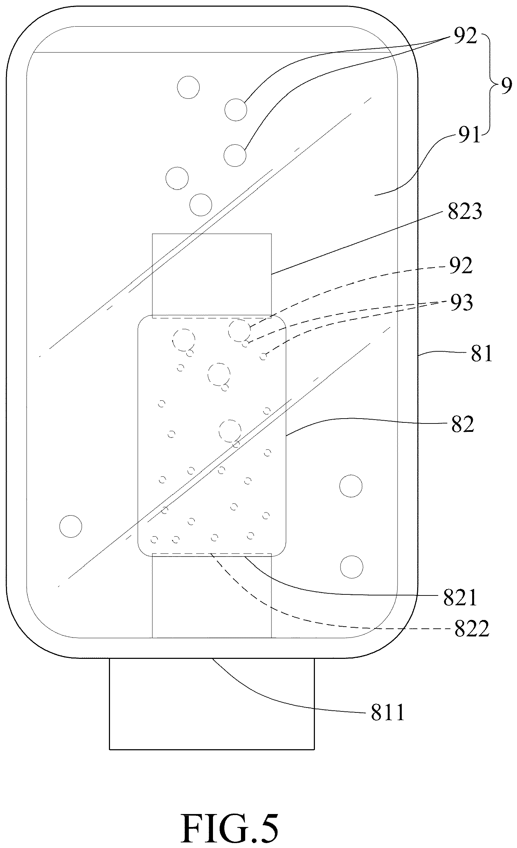

In an exemplary embodiment, as shown in FIG. 5, the step (a) further includes a sub-step (a4') between the sub-step (a4) and the sub-step (a5). In the sub-step (a4'), two covers 823 are used to respectively cover the openings 821 of the second container 82 so as to shield the corresponding hydrophilic membrane filters 822. Each of the covers 823 has a through hole (not shown in the figure) communicating with a respective one of the corresponding openings 821 of the second container 82.

As shown in FIG. 5, the step (c) is inverting the first container 81 so that the opening 811 of the first container 81 faces downward.

As shown in FIG. 5, the first embodiment of the to-be-treated water 91' of the present disclosure substantially fills the first container 81. The opening 811 of the first container 81 is sealed for increasing the partial pressure of hydrogen. However, it should be noted that, if the reduction in the hydrogen is not a major consideration herein, or the reducing level of the hydrogen is within an allowable range, or the opening 811 of the first container 81 is completely sealed, in other embodiments, the step (a5) of inverting the first container 81 may be omitted. In certain embodiments, the to-be-treated water 91' does not necessarily substantially fill the first container 81, and the filling ratio of the to-be-treated water 91' in the first container 81 may also be greater than 90%, and the remaining space in the container 81 may be filled with hydrogen to avoid a decrease in the partial pressure of the hydrogen gas 92 in the to-be-treated water 91' .

According to the present disclosure, a water composition-generating portable product (P) is provided. An embodiment of the water composition-generating portable product (P) includes the second container 82, the silicon material 93, the two hydrophilic membrane filters 822 and the two covers 823. In the water composition-generating portable product (P), the arrangements among the second container 82, the silicon material 93, the two hydrophilic membrane filters 822, and the two covers 823 are as mentioned above and are shown in step (a4') of FIG. 4.

From the aforementioned, it is understood that the use of the silicon material 93 to react with the to-be-treated water 91' can produce the water composition 9 containing the silicic acid and the hydrogen gas 92.

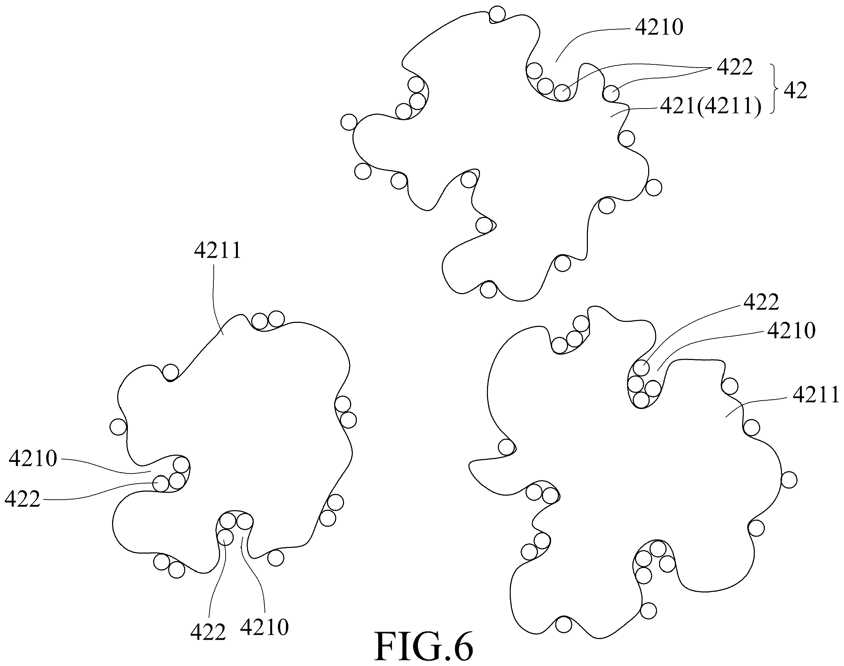

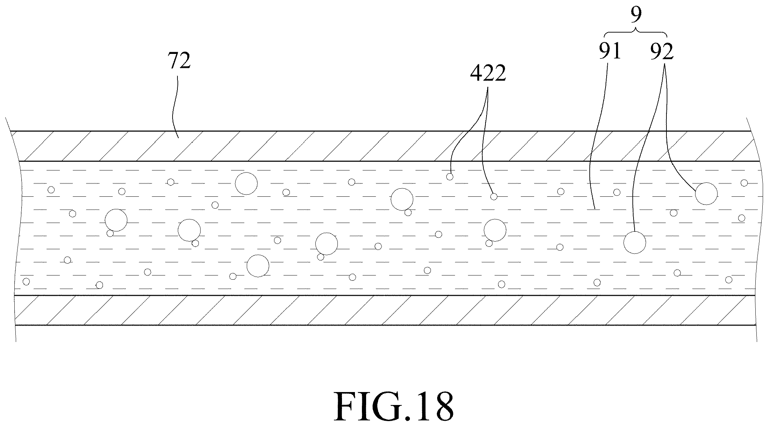

Referring to FIG. 6, a first embodiment of a filter 42 of the present disclosure is adapted for purifying water and producing a water composition 9 containing silicic acid and the hydrogen gas 92 (as shown in FIG. 18). The first embodiment of the filter 42 includes a carrier 421 and a silicon material 422. The carrier 421 may be a porous material, a non-porous material, or the combination thereof. The silicon material 422 is supported (e.g., adsorbed) on a surface of the carrier 421.

The carrier 421 is used for supporting the silicon material 422. As such, any material that can hold and support the silicon material 422 may be used in the present disclosure. Examples of the carrier 421 includes activated carbon, a hollow fiber membrane, bamboo charcoal, porphyritic andesite, quartz sand, fiber, ceramic, and combinations thereof.

The silicon material 422 used in the filter 42 is the aforesaid silicon material 93. In an embodiment, the carrier 421 is an activated carbon 4211 having a plurality of micropores 4210 distributed on a surface thereof, and the silicon material 422 is the nano silicon having an average diameter ranging between 100 nm and 250 nm. A portion of the silicon material 422 is disposed in the micropores 4210 of the activated carbon 4211.

According to the invention, based on the weight of the filter 42, the carrier 41 is present in an amount less than or equal to 90 wt%, and the silicon material 422 is present in an amount greater than or equal to 10 wt%, and thus, the filter 42 can simultaneously has the functions of water purification and generation of the water composition 9.. In another exemplary embodiment, the silicon material 422 is present in an amount between 10 wt% and 40 wt%. In yet another exemplary embodiment, the silicon material 422 is present in an amount between 15 wt% and 30 wt%. In a further another exemplary embodiment, the silicone material 422 is present in an amount not smaller than 20 wt%, and the carrier 421 is present in an amount not greater than 80 wt%.

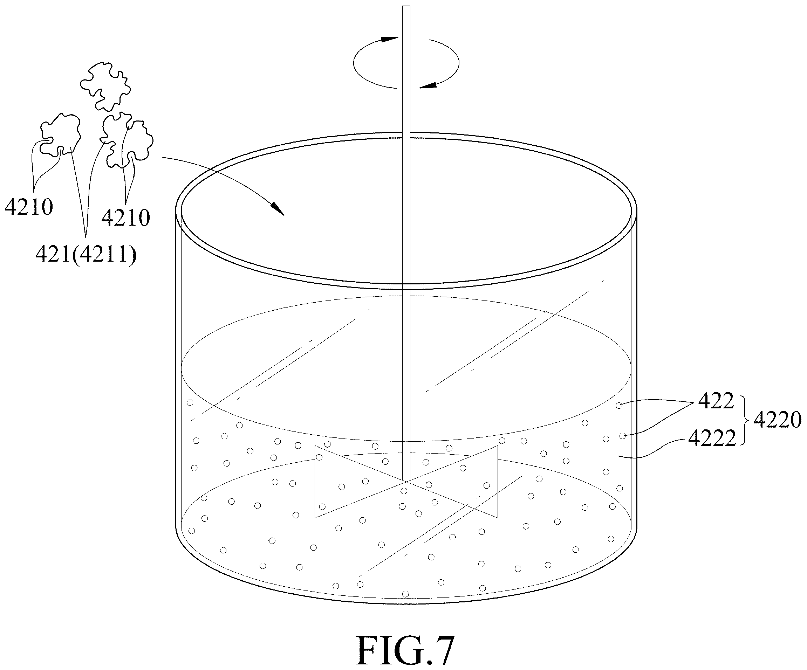

Referring to FIG. 7, a method for making the first embodiment of the filter 42 of the present disclosure includes: adding a silicon slurry 4220 (containing a solvent 4222 (e.g., alcohol) and the silicon material 422 (e.g., nano silicon)) into a container having stirring function, adding the carrier 421 (e.g., the activated carbon 4211 with micropores 4210) into the container, and stirring the mixture of the silicon slurry 4220 and the carrier 421 so that the silicon material 422 can enter into the micropores 4210 of the activated carbon 4211 through turbulent currents generated by stirring and then is adsorbed on the activated carbon 4211. The solvent 4222 is then removed by e.g., heating, so as to obtain the first embodiment of the filter 42.

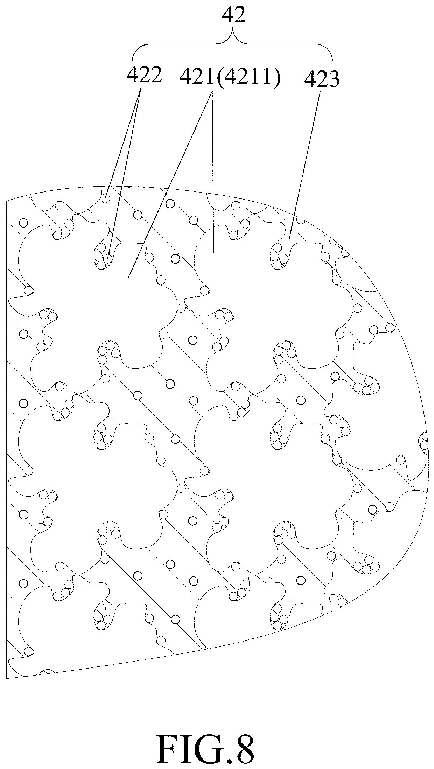

Referring to FIG. 8, a second embodiment of the filter 42 of the present disclosure is similar to the first embodiment except that the filter 42 of the second embodiment further includes a binder 423. The binder 423 binds the silicon material 422 (e.g., the nano silicon) and the carrier 421 (e.g., the activated carbon 4211), so that a portion of the silicon material 422 is adsorbed and bound onto the carrier 421. In an embodiment of this disclosure, the silicon material 422 is the nano silicon, and the carrier 421 is the activated carbon 4211, and the binder 423 and the activated carbon 4211 together constitute a sintered activated carbon. To be specific, the second embodiment of the filter 42 is made by mixing the activated carbon 4211, the nano silicon (i.e., the silicon material 422) and the binder 423 to obtain a mixture, hot pressing the mixture in a mold to form a green body, and finally sintering the green body into the filter 42. It is worth mentioning that after sintering, the binder 423 is formed with a plurality of holes (not shown in the figure) to allow the water 9 to flow therethrough.

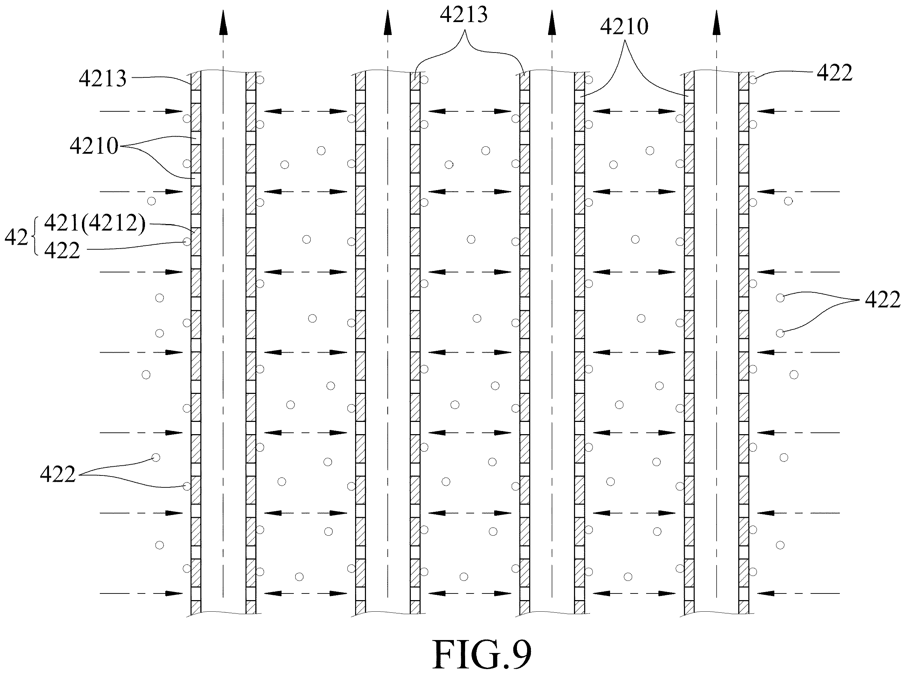

Referring to FIG. 9, a third embodiment of the filter 42 of the present disclosure is similar to the first embodiment. The difference resides in that, the carrier 421 of the third embodiment is a hollow fiber membrane 4212 having a hollow fiber 4213 formed with a plurality of through holes 4210 thereon. The average diameter of the through holes 4210 ranges between 10 nm and 100 nm. The silicon material 422 (in this embodiment, the silicon material is nano silicon) is supported onto a surface of the hollow fiber membrane 4212. In certain embodiments, the hollow fiber membrane 4212 may include a plurality of hollow fibers 4213, each of which is being formed with the through holes 4210 (see FIG. 9).

The method for making the third embodiment of the filter 42 of the present disclosure includes: mixing the silicon material 422 with a fluid (e.g., water), directing the silicon material 422 by a flow direction of the fluid to the hollow fiber membrane 4212 so that the silicon material 422 is supported onto the surface of the hollow fiber membrane 4212. The fluid also flows through the through holes 4210 and travels toward an outlet of the hollow fiber membrane 4212. In certain embodiments, the silicon material 422 may be bound onto the hollow fiber membrane 4212 by virtue of a binder 423. Otherwise, the silicon material 422 may be added during production of the hollow fiber membrane 4212.

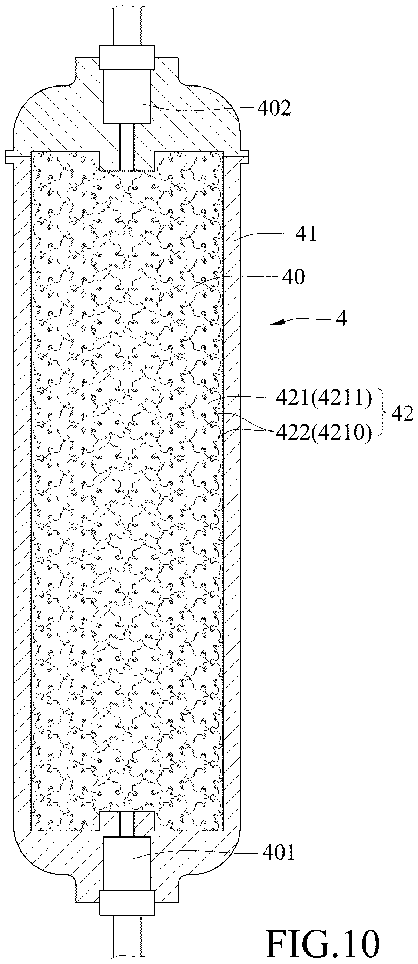

Referring to FIG. 10, a first embodiment of a filter assembly 4 of the present disclosure is adapted for purifying water and producing the water composition 9 containing the silicic acid and the hydrogen gas 92. The filter assembly 4 includes a housing 41 and a filter 42 as described in any of the above embodiments of the filter 42 (e.g., the filter 42 shown in FIGS. 6, 8 and 9). The housing 41 defines a receiving space 40, and has an inlet 401 and an outlet 402 which are disposed at two opposite sides of the housing 41 and which are in fluid communication with the receiving space 40. The filter 42 is received in the receiving space 40 of the housing 41. In certain embodiments, the inlet 401 is disposed below the outlet 402 in an operating state.

In certain embodiments, the filter assembly 4 may further include a water flow-dispersion unit and a blocking unit. The water flow-dispersion unit is located in the receiving space 40 of the housing 41 and is adjacent to the inlet 401, and the blocking unit is located in the receiving space 40 of the housing 41 and is adjacent to the outlet 402.

As shown in FIG. 10, in this embodiment, the filter 42 is the first embodiment of the filter 42 shown in FIG. 6, and the carrier 421 is the activated carbon 4211 distributed uniformly in the receiving space 40 of the housing 41, and the silicon material 422 is the nano silicon. With the micropores 4210 in which a portion of the silicon material 422 is deposited, the contact area between the water 91 and the silicon material 422 may be increased, thereby increasing the hydrogen content in the water composition 9.

Moreover, in certain embodiments, the filter assembly 4 is vertically disposed (see FIGs. 10 to 16) such that the outlet 402 is located at the opposite end of the housing 51 which is at a higher level than that of the other opposite end of the housing 51. Thus, the loss of the silicon material 422 along with the flowing of the water may be reduced.

It is noted that, in certain embodiments, the housing 41 of filter assembly 4 may be filled with only the silicon material 422 to produce the water composition 9 containing the silicic acid and the hydrogen gas 92.

Apart from directly using the first embodiment of the filter 42, the first embodiment of the filter assembly 4 can also be so prepared that a first portion of the carrier 421 (i.e., the activated carbon 4211), the silicon material 422 and a second portion of the carrier 421 (i.e., the activated carbon 4211) are sequentially disposed in the receiving space 40 of the housing 41 of the filter assembly 4 in a direction from the inlet 401 toward the outlet 402 of the housing 41. In use, when to-be-treated water (not shown in this figure) is introduced into the housing 41 from the inlet 401, the first portion of the activated carbon 4211 disposed proximate to the inlet 401 can uniformly disperse the to-be-treated water . The silicon material 422 is moved along the water flow direction (F) and is then supported onto the carrier 421. The second portion of the activated carbon 4211 disposed proximate to the outlet 402 can reduce the impact of water current and block the silicon material 422 from flowing out of the housing 41. As such, in the first embodiment of the filter assembly 4, the first portion of the carrier 421 disposed proximate to the inlet 401 is used as the water flow-dispersion unit, and the second portion of the carrier 421 disposed proximate to the outlet 402 is used as the blocking unit.

The purpose of the water flow-dispersion unit is to prevent water from accumulating at the inlet 401, and to evenly distribute water in the receiving space 40 of the housing 41. As such, the water flow-dispersion unit suitable for this disclosure is not limited to the activated carbon 4211 mentioned above, and may be glass beads, a partition with holes, a non-woven fabric, and the like. Moreover, the purpose of the blocking unit is to reduce the probability that the silicon material 422 is being carried out of the receiving space 40 with the flow water. Therefore, the blocking unit suitable for the present disclosure is not limited to the above-mentioned activated carbon 4211. Othermaterials, such as bamboo charcoal, porphyritic andesite (also known as medical stone), hollow fiber membrane, fiber or quartz sand, etc. that can block the silicon material 422 at the location proximate to the outlet 402 are also suitable.

Furthermore, the filter assembly 4 may further includes a material (e.g., medical stone, etc.) that is capable of releasing trace elements (e.g., rubidium, strontium, selenium, etc.) and/or other minerals (e.g., potassium, etc.). Thus, the water composition 9 may further includes the trace elements and other minerals in addition to the silicic acid and the hydrogen gas 92.

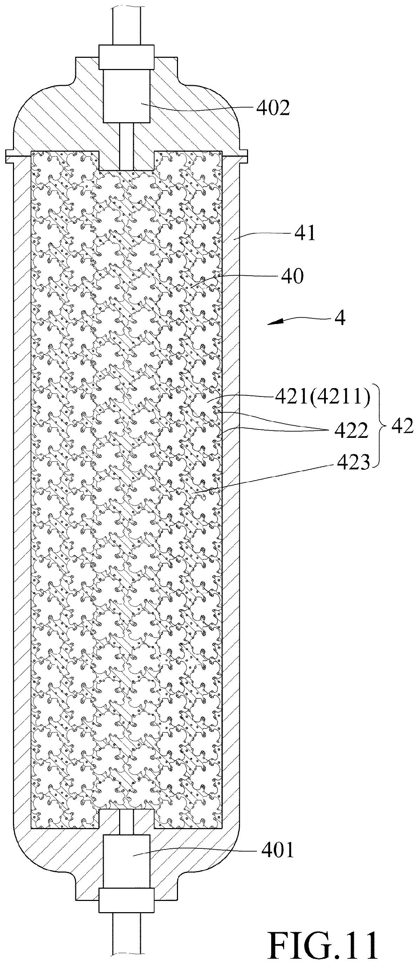

Referring to FIG. 11, the second embodiment of the filter assembly 4 of the present disclosure is similar to the first embodiment except that the filter 42 used in this embodiment is the one shown in FIG. 8 (i.e., the second embodiment of the filter 42 which includes the binder 423) . With the binder 423 which binding the carrier 421 (e.g., activated carbon 4211) and the silicon material 422, the silicon material 422 can be more firmly supported on the carrier 421 so as to prevent the silicon material 422 from being flushed with water and flowing out of the housing 41.

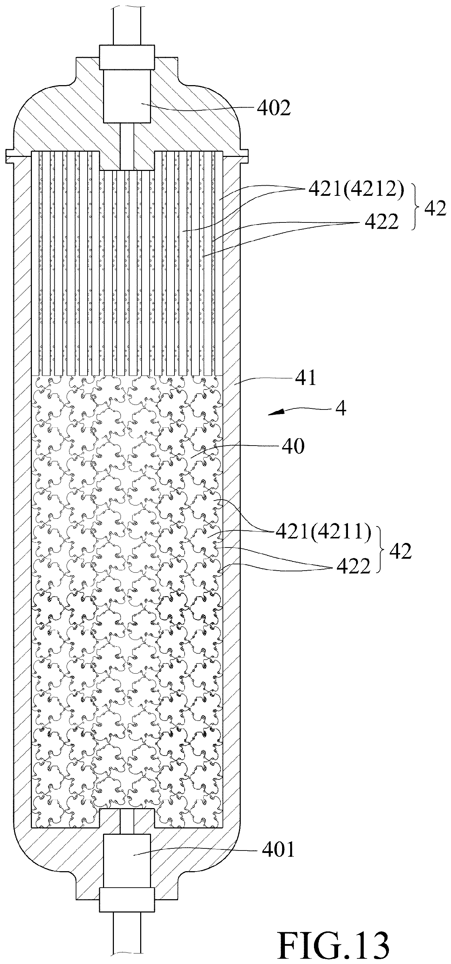

Referring to FIG. 13, a third embodiment of the filter assembly 4 of the present disclosure is substantially the same as the first embodiment except that the carrier 421 is the combination of the activated carbon 4211 and the hollow fiber membrane 4212, and the silicon material 422 is the nano silicon. That is, the third embodiment of the filter assembly 4 includes the first embodiment of the filter 42 and the third embodiment of the filter 42. In this embodiment, the activated carbon 4211 is distributed evenly in a part of the receiving space 40 of the housing 41, and is disposed proximate to the inlet 401 relative to the hollow fiber membrane filters 4212, and the hollow fiber membrane 4212 is disposed in another part of the receiving space 40 of the housing 41, and is disposed proximate to the outlet 402 relative to the activated carbon 4211.

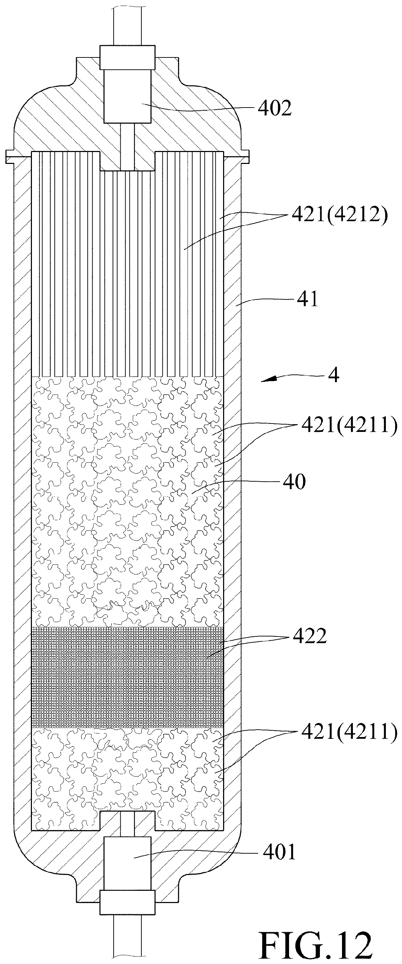

Apart from directly using the first and third embodiments of the filter 42 (in which the silicon material 422 has been distributed in the carrier 421), as shown in FIG. 12, the third embodiment of the filter assembly 4 of the present disclosure can also be so prepared that, a first portion of the activated carbon 4211), the silicon material 422, a second portion of the activated carbon 4211, and the hollow fiber membrane 4212 are sequentially disposed from the inlet 401 toward the outlet 402 of the housing 41. In use, the to-be-treated water is introduced into the housing 41 through the inlet 401 (not shown in this figure), the to-be-treated water can substantially fill the receiving space 40, and drive the silicon material 422 to move along the water flow direction (F). A portion of the silicon material 422 is deposited in the micropores 4210 of the activated carbon 4211 such that the contact area between the to-be-treated water and the silicon material 422 may be increased, thereby increasing the hydrogen content in the water composition 9. Moreover, the silicon material 422 not deposited in the micropores 4210 of the activated carbon 4211 can still be brought to the hollow fiber membrane 4212 along the water flow direction (F) and is adsorbed onto the surface of the hollow fiber membrane 4212. Thus, the water composition 9 continues to react with the silicon material 422 in the hollow fiber membrane 4212, and an aeration effect will be occurred here so as to further increase the amount of dissolved hydrogen gas 92. In addition, the hollow fiber membrane 4212 can also block the aggregated and precipitated silicic acid generated due to supersaturation. Since the to-be-treated water is continuously introduced into the filter assembly 4, the aggregated and precipitated silicic acid may be further dissolved.

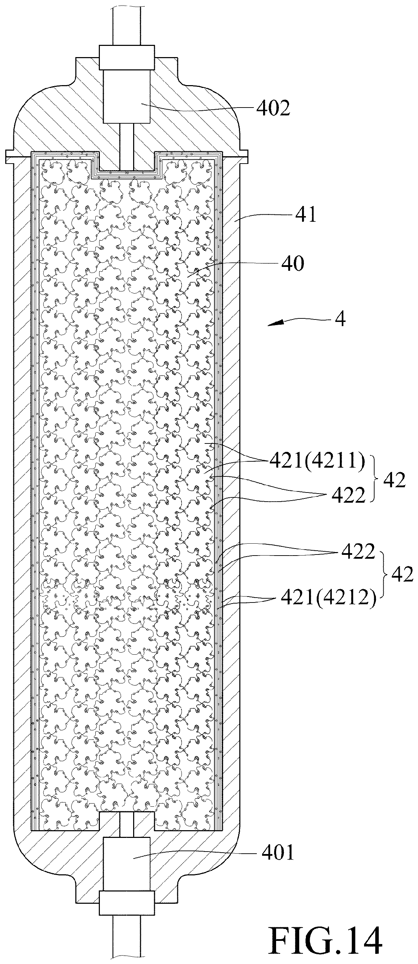

Referring to FIG. 14, a fourth embodiment of the filter assembly 4 of the present disclosure is substantially the same as the third embodiment. The difference resides in the arrangement of the filter 42 in the receiving space 40 of the housing 41 of the filter assembly 4. Similarly, the carrier 421 is the combination of the activated carbon 4211 and the hollow fiber membrane 4212, and the silicon material 422 is the nano silicon. The activated carbon 4211 is distributed evenly in the receiving space 40 of the housing 41, and the hollow fiber membrane 4212 is located in the receiving space of 40 of the housing 41 to surround the activated carbon 4211. In this embodiment, the activated carbon 4211 is disposed proximate to the inlet 401, and the hollow fiber membrane 4212 is disposed proximate to the outlet 402. In other words, the receiving space 40 proximate to the inlet 401 is filled with the activated carbon 4211, and the receiving space 40 proximate to the outlet 402 is filled with the hollow fiber membrane 4212.

To be specific, in this embodiment, the receiving space 40 is filled with the first embodiment of the filter 42 (i.e., the silicon material 422 deposited in the micropores 4210 of the activated carbon 4211) and the third embodiment of the filter 42 (i.e., the silicon material 422 is adsorbed onto the surfaces of the hollow fiber membrane 4212), and the third embodiment of the filter 42 is disposed to surround the first embodiment of the filter 42.

Similar to the third embodiment of the filter assembly 4 shown in FIG. 12, in this embodiment, the silicon material 422 may be separately disposed in the receiving space 40 of the housing 41 from the activated carbon 4211 and the hollow fiber membrane 4212. When the to-be-treated water (not shown in FIG. 14) is introduced into the housing 41 from the inlet 401, the silicon material 422 can flow along the water flow direction (F) to pass through and thus be supported on the activated carbon 4211 and the hollow fiber membrane filter 4212.

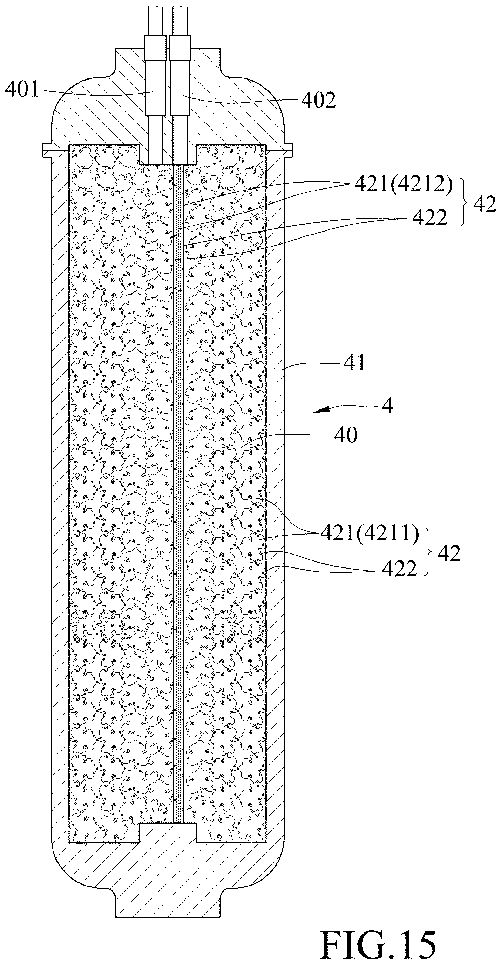

Referring to FIG. 15, a fifth embodiment of the filter assembly 4 of the present disclosure is substantially the same as the fourth embodiment. The difference resides in the arrangement of the filter 42 in the receiving space 40 of the housing 41 of the filter assembly 4, and the positional relationship of the inlet 401 and the outlet 402. Similarly, the carrier 421 is the combination of the activated carbon 4211 and the hollow fiber membrane 4212, and the silicon material 422 is the nano silicon. In this embodiment, the inlet 401 and the outlet 402 are disposed at the same side of the housing 41. The hollow fiber membrane 4212 is surrounded by the activated carbon 4211. In other words, the hollow fiber membrane 4212 is disposed in the center of the receiving space 40, while the activated carbon 4211 surrounds the hollow fiber membrane 4212. The activated carbon 4211 is disposed proximate to the inlet 401, and the hollow fiber membrane filters 4212 is disposed proximate to the outlet 402. In other words, the receiving space 40 adjacent to the inlet 401 is filled with the activated carbon 4211, and the receiving space 40 adjacent to the outlet 402 is filled with the hollow fiber membrane 4212.

In use, to-be-treated water (not shown in FIG. 15) is introduced into the housing 41 from the inlet 401 and passes through the activated carbon 4211, and then reacts with the silicon material 422 supported on the activated carbon 4211 to generate the silicic acid and the hydrogen gas 92. The water composition 9 containing the dissolved silicic acid and the hydrogen gas 92 flow into the through holes 4210 of the hollow fiber membrane 4212 and then flow out of the housing 41 through the outlet 402. Some of the silicon material 422 can be brought to the position where the hollow fiber membrane 4212 is located along the water flow direction (F) and is supported on the hollow fiber membrane 4212.

It should be noted that, in the first to fourth embodiments of the filter assembly 4, the inlet 401 and the outlet 402 may be disposed at the same side of the housing 41.

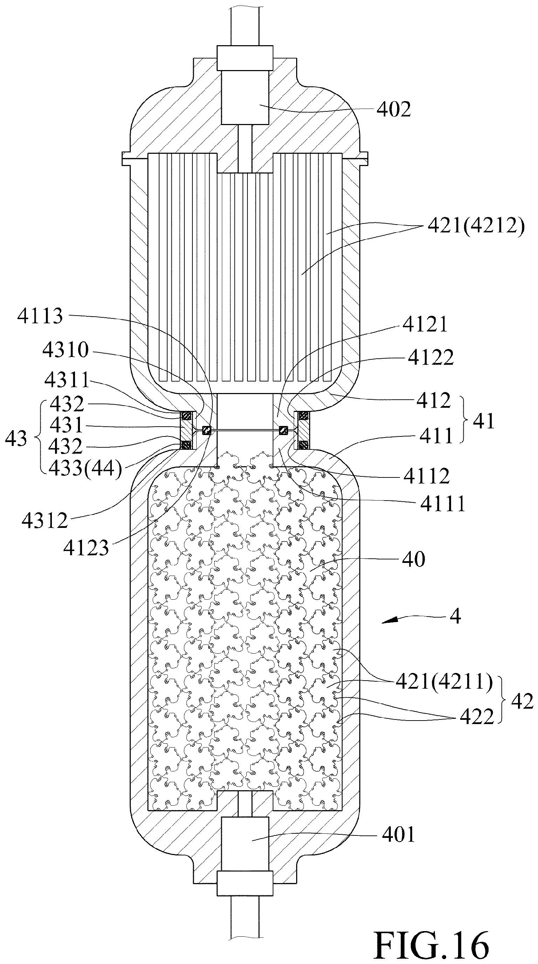

Referring to FIG. 16, a sixth embodiment of the filter assembly 4 of the present disclosure is similar to the third embodiment except that the sixth embodiment of the filter assembly 4 further includes a detachable connecting member 43, and has differences in detailed structure of the housing 41 and the arrangement of the filter 42.

Specifically, the housing 41 has a first member 411 and a second member 412 detachable from each other. The first member 411 and the second member 412 respectively include the inlet 401 and the outlet 402 of the housing 41. Each of the first member 411 and the second member 412 has a connecting section 4111, 4121. In use, the connecting sections 4111, 4121 face each other and are in spatial communication with each other . The detachable connecting member 43 is connected to and is disposed between the first member 411 and the second member 412, and the receiving space 40 of the housing 41 is defined cooperatively by the first member 411 and the second member 412. In this embodiment, the carrier 421 is the combination of the activated carbon 4211 and the hollow fiber membrane 4212, and the silicon material 422 is the nano silicon. In this embodiment, the activated carbon 4211 is located in the first member 411 of the housing 41, and the hollow fiber membrane 4212 is located in the second member 412 of the housing 41. In other words, the first embodiment of the filter 42 (i.e., the silicon material 422 (such as the nano silicon) deposited in the micropores 4210 of the activated carbon 4211) is filled in the first member 411, while the third embodiment of the filter 42 (i.e., the silicon material 422 (such as the nano silicon) is supported on the surface of the hollow fiber membrane 4212) is filled in the second member 412.

In this embodiment, the detachable connecting member 43 is a rotary connection assembly. The connecting section 4111, 4121 of each of the first member 411 and the second member 412 is formed with an external thread 4112, 4122 and a loop groove 4113, 4123 that is in the shape of a loop. The loop groove 4113 of the first member 411 and the loop groove 4123 of the second member 412 face each other to define an accommodating space 44. The rotary connection assembly of the detachable connecting member 43 includes a nut 431 formed with an internal thread 4310, an elastic inner seal ring 433 and a pair of elastic outer seal rings 432. The nut 431 is formed with an upper recess 4311 and a lower recess 4312 respectively recessed in two opposite ends of the nut. The outer seal rings 432 are respectively disposed in the upper recess 4311 and the lower recess 4312. The inner seal ring 433 is disposed in the accommodating space 44 defined by the loop grooves 4113, 4123 of the first member 411 and the second member 412. To be specific, the external threads 4112, 4122 of the first member 411 and the second member 412 are threadedly connected to the internal thread 4310 of the nuts 431 so as to connect the first member 411 and the second member 412 and define the receiving space 40 of the housing 41. The inner seal ring 433 and the outer seal rings 432 seal the receiving space 40 to avoid leakage of to-be-treated water (not shown in FIG. 16) from the receiving space 40.

The first and second members 411, 412 may be connected with the detachable connecting member 43 by virtue of other connecting manner. In certain embodiments, the first and second members 411, 412 are inserted fittingly in the detachable connecting member 43.

FIG. 16 shows a state where the water has yet to be introduced into the second member 412 of the housing 41. Once the sixth embodiment of the filter assembly 4 is put to use, the silicon material 422 can be carried to the second member 412 along with water flow direction (F) so as to be supported onto the surface of the hollow fiber membrane 4212.

In this embodiment, the filter assembly 4 is a separable filter assembly. Briefly, when the silicon material 422 of the first embodiment of the filter 42 filled in the first member 411 is unable to be reacted with water to produce the silicic acid and the hydrogen gas 92, the nut 431 of the rotational connection assembly is loosened to detach the first member 411 from the second member 412, so that the filter 42 in the first member 411 can be replaced. After replacement, the nut 431 of the rotational connection assembly is tightened to connect the connecting segment 4111 of the first member 411 and the connecting segment 4121 of the second member 412. In this situation, the housing 41 of the filter assembly 4 can be repeatedly used so as to achieve cost-saving.

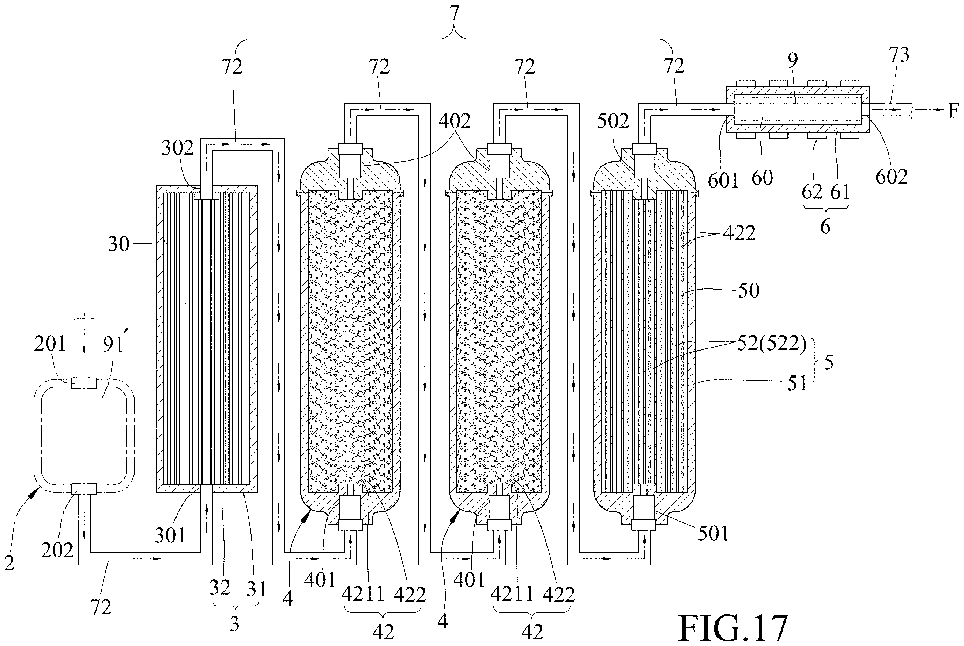

Referring to FIGS. 17 and 18, a first embodiment of a water purification system of the present disclosure is used to produce the water composition 9 containing the silicic acid (not shown in the figure) and the hydrogen gas 92. The water purification system includes at least one of the aforesaid filter assemblies 4 (i.e., the first to sixth embodiments of the filter assemblies 4) as a first filter assembly and a pipeline unit 7. The water purification system may optionally further include a degassing unit 3, at least one second filter assembly 5, and/or an ultraviolet light sterilization unit 6. The water purification system may be connected to a water-generating device 2 that can generate to-be-treated water 91' to be fed into and treated by the water purification system. The water-generating device 2 includes an inlet 201 and an outlet 202, and may be a commercially available reverse osmosis (R.O.) device, or any other type of water purification equipment. Thus, the to-be-treated water 91' to be fed into the water purification system of this disclosure is filtered water. However, natural water, underground water or other unfiltered water may be used directly as well. It should be noted that, under appropriate circumstance (e.g., the R.O. device is used as the water-generating device 2), the water purification system of the present disclosure may merely include the aforesaid filter assembly 4 of the present disclosure.

In this embodiment, the degassing unit 3 is disposed upstream of the first filter assembly 4 in the water flow direction (F), and is connected to the first filter assembly 4 through a transport pipe 72 of the pipeline unit 7. Specifically, the degassing unit 3 includes a casing 31 defining a receiving space 30 therein and a degassing member 32 received in the receiving space 30. The degassing unit 3 is used to remove gases (e.g., CO₂, O₂, N₂, etc.) originally dissolved in the to-be-treated water 91' so as to enhance the amount of hydrogen to be dissolved in the water 91 of the water composition 9. The degassing unit 3 suitable for use in the water purification system of this disclosure may be a filter-type air extractor, or any other type of filter capable of adsorbing gas.

In certain embodiments, the second filter assembly 5 is disposed downstream of the first filter assembly 4 along the water flow direction (F) . The second filter assembly 5 includes a housing 51 defined with a receiving space 50, and a porous material 52 filled in the receiving space 50. The housing 51 of the second filter assembly 5 includes an inlet 501 and an outlet 502 disposed at two opposite ends of the housing 51 and in fluid communication with the receiving space 50. The porous material 52 of the second filter assembly 5 may be activated carbon 521, a hollow fiber membrane 522 or the combination thereof. The first filter assembly 4 and the second filter assembly 5 are fluidly connected each other through a transport pipe 72 of the pipeline unit 7.

The ultraviolet sterilizing unit 6 is disposed downstream of the first filter assembly 4 along the water flow direction (F), and is fluidly connected to the first filter assembly 4 via the pipeline unit 7 for sterilizing the water composition 9. In this embodiment, the ultraviolet sterilizing unit 6 is disposed downstream of the first filter assembly 4 and the second filter assembly 5. Specifically, the ultraviolet light sterilization unit 6 includes a housing 61 defining a receiving space 60, and an ultraviolet lamp unit 62. The housing 61 includes an inlet 601 and an outlet 602 disposed at two opposite ends of the housing 61 and in fluid communication with the receiving space 60. In this embodiment, the ultraviolet lamp unit 62 includes a plurality of UV lamps arranged in a direction from the inlet 601 to the outlet 602.

Referring to FIG. 17, the inlet 201 of the water-generating device 2 is connected to an inlet pipe 71. The water composition 9 flows out of the ultraviolet light sterilization unit 6 through an outlet pipe 73.

In this embodiment, the water purification system includes the degassing unit 3, two of the first filter assemblies 4, the second filter assembly 5 and the ultraviolet light sterilization unit 6 sequentially arranged along the water flow direction (F) . The number of the transport pipe 72 of the pipeline unit 7 is five. In this embodiment, each of the first filter assemblies 4 is the first embodiment of the filter assembly 4 shown in FIG. 10. The second filter assembly 5 is disposed in an upright position, and the porous material 52 of the second filter assembly 5 is the hollow fiber membrane 522.

To be specific, the inlet pipe 71 is connected to the inlet 201 of the water unit 2, and the five transport pipes 72 respectively connect the outlet 202 of the water-generating device 2 and an inlet 301 of the degassing unit 3, an outlet 302 of the degassing unit 3 and an inlet 401 of one of the two first filter assemblies 4 immediately connected to the degassing unit 3 (hereinafter referred to as upstream first filter assembly), an outlet 402 of the upstream first filter assembly 4 and an inlet 401 of the other one of the first filter assemblies 4 disposed downstream of the upstream first filter assembly (hereinafter referred to as downstream first filter assembly), an outlet 402 of the downstream first filter assembly 4 and the inlet 501 of the second filter assembly 5, and the outlet 502 of the second filter assembly 5 and the inlet 601 of the ultraviolet light sterilization unit 6. The outlet pipe 73 is to be connected to the outlet 602 of the ultraviolet light sterilization unit 6.

Furthermore, when the water composition 9 moves along the water flow direction (F), disturbance phenomenon might occur naturally. This disturbance phenomenon may gradually result in accumulation of gas that may affect the flow of the water composition 9. Thus, the reason for placing the second filter assembly 5 in an upright position is for the hollow fiber membrane 522 in the receiving space 50 of the housing 51 to be arranged in the upright position, so that the gas accumulating in the receiving space 50 of the second filter assembly 5 can be easily expelled out from the outlet 502 to the transport pipe 72.

Referring again to FIG. 17 and FIG. 18, the first embodiment of the water purification system may be used in a method for producing a second embodiment of the water composition 9 of the present disclosure. The method for producing the second embodiment of the water composition 9 includes the steps of: delivering the to-be-treated water 91' produced by the water-generating device 2 to the degassing unit 3 to remove CO₂, O₂ and N₂ in the to-be-treated water 91', and introducing the to-be-treated water 91' that has been degassed to flow through the pipeline unit 7 from the outlet 302 of the degassing unit 3 to the upstream first filter assembly 4 so as to react the to-be-treated water 91' with the silicon material 422 supported on the carrier 421 (e.g., activated carbon 4211), thereby generating the water composition 9 containing the silicic acid and the hydrogen gas 92; delivering the water composition 9 (containing the water 91, the silicic acid and the hydrogen gas 92) and a part of the silicon material 422 from the outlet 402 of the upstream first filter assembly 4 through the transport pipe 72 of the pipeline unit 7 into the downstream first filter assembly 4, so that the water composition 9 continues to react with the silicon material 422 of the downstream first filter assembly 4 to further generate the silicic acid and the hydrogen gas 92 so as to increase the content of the dissolved hydrogen gas 92 of the water composition 9, and then delivering the water composition 9 from the outlet 402 of the downstream first filter assembly 4 through the pipeline unit 7 into the second filter assembly 5, in which the water composition 9 flows through the hollow fiber membrane 522 of the second filter assembly 5 along the water flow direction (F) so that the silicon material 422 released from the first filter assemblies 4 is partially or completely adsorbed on the hollow fiber membrane 522, and introducing the water composition 9 from the outlet 502 of the second filter assembly 5 along the water flow direction (F) into the receiving space 60 of the ultraviolet light sterilization unit 6 to sterilize the water composition 9 using the ultraviolet lamp unit 62, and then delivering the water composition 9 from the outlet 602 of the ultraviolet light sterilization unit 6 to the outlet pipe 73.

In the second embodiment of the water composition 9 produced by the aforesaid, the concentration of the silicic acid dissolved in the water 91 is between 40 mg/L and the saturation concentration of the silicic acid, and the ORP of the water composition 9 is below -500 mV. In certain embodiments, the ORP of the second embodiment of the water composition 9 is less than or equal to -650 mV.

It is worth mentioning that the number of the first filter assembly 4 may vary. The more the number of the first filter assembly 4, the lower the ORP value will be. Furthermore, if the downstream first filter assembly 4 is the one having the hollow fiber membrane 4212 (i.e., the third to sixth embodiments of the filter assemblies 4 respectively shown in FIGS. 13 to 16), the second filter assembly 5 can be omitted.

Apart from being used as drinking water, the water composition 9 of the present disclosure can be added into an article that will be applied to plants or animals so as to slow down the oxidation rate of the article, the plants and the animals, or to facilitate formation of the connective tissue in the animals. The article may be one that can be externally or internally applied to the animals. Examples of the article may include, but are not limited to, a skin care product or a beverage . The animal may be a vertebrate, but is not limited thereto . Examples of the skin care product may include, but are not limited to toning lotions, moisturizing lotions, moisturizing creams, essences, whitening lotions, whitening milks, whitening creams, etc. In addition, examples of the beverage includes e.g., apple juice. The water composition 9 can be used to enhance the preservation of the beverage. The water composition 9 of this disclosure can also be used in any other application where water is needed except for applications where hydrogen and/or silicic acid cannot be used.

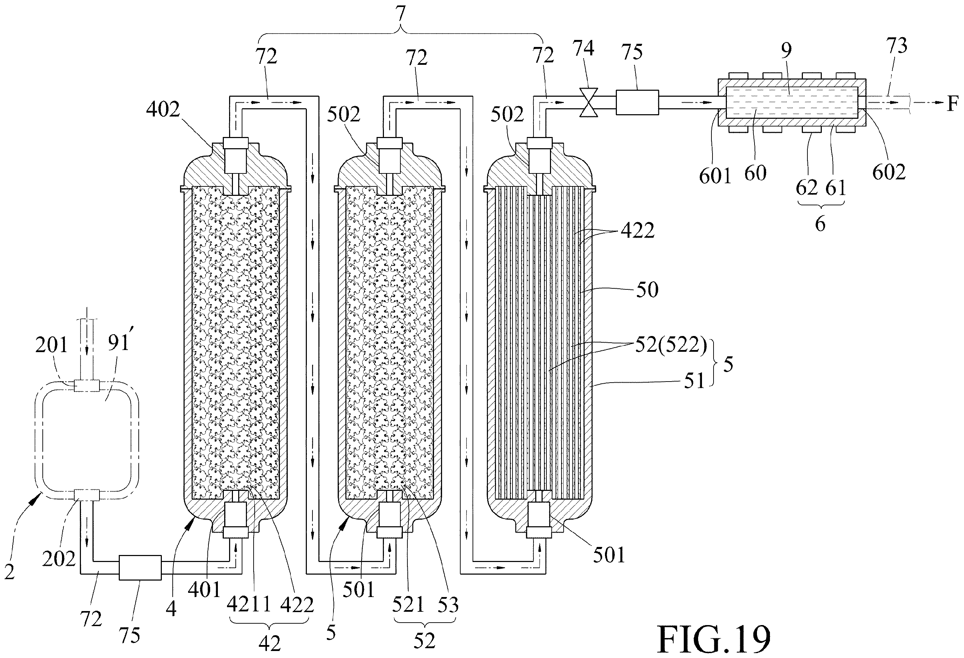

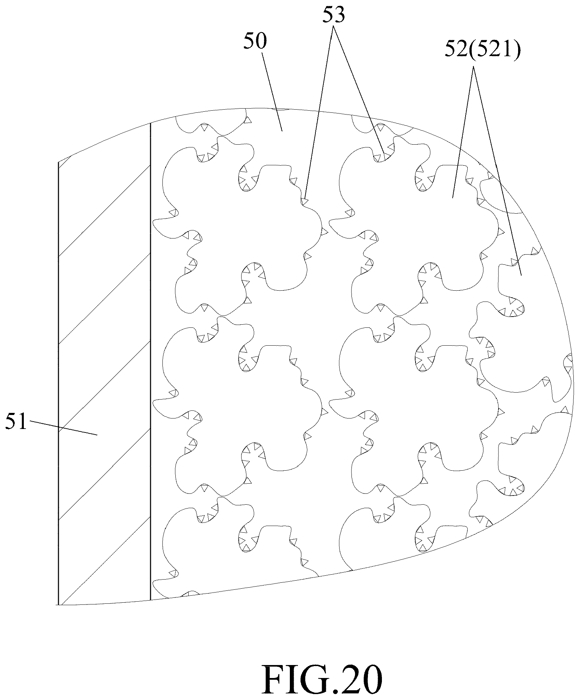

Referring to FIG. 19 and FIG. 20, the second embodiment of the water purification system of the present disclosure is substantially the same as the first embodiment. The differences reside in that the second embodiment of the water purification system does not include the degassing unit 3, but further includes a discharge unit 74 and two measuring units 75. Moreover, the second embodiment of the water purification system includes one first filter assembly 4 and a plurality of the second filter assemblies 5. In this embodiment, the number of the second filter assembly 5 is two (the upstream second filter assembly and the downstream second filter assembly disposed downstream of the upstream second filter assembly), and thus the pipeline unit 7 has four of the transport pipes 72. The porous materials 52 filled in the receiving spaces 50 of the upstream and downstream second filter assemblies 5 are respectively the activated carbon 521 and the hollow fiber membrane 522. The upstream second filter assembly 5 further includes a nanometer silver 53 supported on the activated carbon 521.

Referring to FIG. 19, the discharge unit 74 is disposed downstream of the downstream second filter assembly 5, and is operationally connected to the pipeline unit 7. Specifically, the discharge unit 74 is a pressure relief valve operationally and fluidly connected to the transport pipe 72 and is disposed downstream of the outlet 502 of the downstream second filter assembly 5 and upstream of the inlet 601 of the ultraviolet light sterilization unit 6 for discharging a liquid, gas, or the combination thereof from the pipeline unit 7 . During production of the water composition 9 in the first filter assembly 4 and the second filter assemblies 5, gas will be continuously generated and accumulated due to disturbance of the water 91, causing undesired high pressure in the transport pipes 72 that can adversely affect the water flow. The discharge unit 74 (e.g., the pressure relief valve) included in the second embodiment of the water purification system could release the undesired high pressure in the transport pipes 72 so as to enhance the liquid flow.

In this embodiment, the two measuring units 75 are respectively mounted upstream and downstream of the first filter assembly 4 along the water flow direction (F) . Specifically, one of the measuring units 75 disposed upstream of the first filter assembly 4 (hereinafter referred to as upstream measuring unit) is operationally connected to the transport pipe 72 that connects the outlet 202 of the water-generating device 2 and the inlet 401 of the first filter assembly 4, whereas the other one of the measuring units 75 disposed downstream of the first filter assembly 4 (hereinafter referred to as downstream measuring unit) is operationally connected to the transport pipe 72 that connects the outlet 502 of the second filter assembly 5 and the inlet 601 of the ultraviolet light sterilization unit 6, and is disposed downstream of the discharge unit 74. The measuring units 75 are used to measure the amount of the total dissolved solid (TDS) in the fluid. In this embodiment, the measuring units 75 are TDS meters. When the to-be-treated water 91' is R.O. water, the amount of total dissolved solids (hereinafter referred to as Vₜ) measured by the downstream measuring unit 75 will be greater than the amount of total dissolved solids (hereinafter referred to as V₀) measured by the upstream measuring unit 75. Once the difference (ΔV) between Vₜ and V₀ is smaller than a predetermined value, it indicates that the amount of the silicic acid in the water composition 9 has gradually decreased, and replacement of the filter 42 of the first filter assembly 4 is needed.

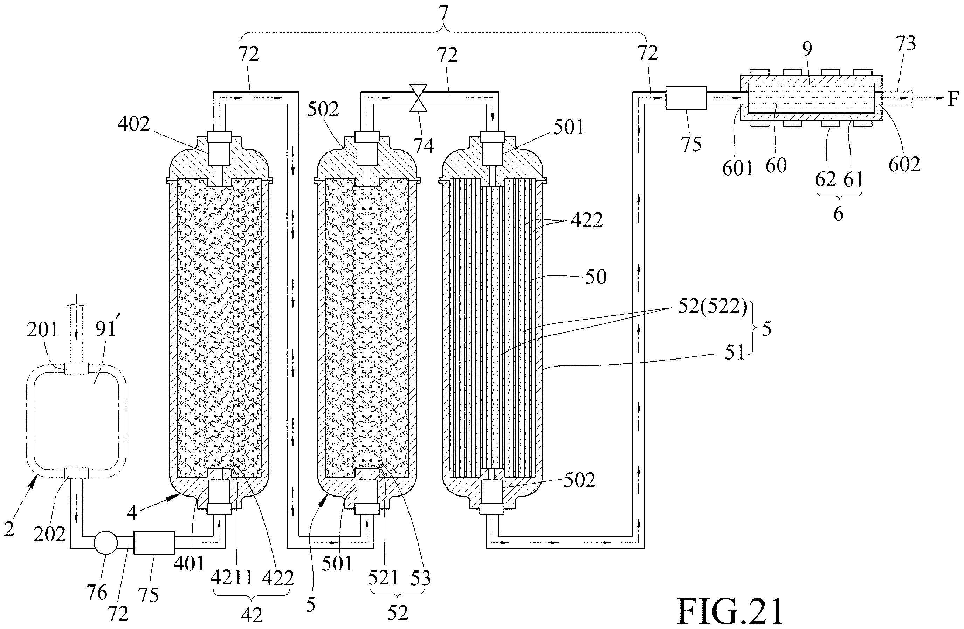

Referring to FIG. 21, the third embodiment of the water purification system of this disclosure is similar to the second embodiment. The difference resides in that the water purification system of the third embodiment further includes a flow meter 76, and the discharge unit 74 is disposed between the upstream second filter assembly 5 and the downstream second filter assembly 5. The flow meter 76 is disposed upstream of the first filter assembly 4 along the water flow direction (F).

In this embodiment, the discharge unit 74 (e.g., gas discharge valve) is operationally connected to the transport pipe 72 connecting the inlet 501 of the downstream second filter assembly 5 and the outlet 502 of the upstream second filter assembly 5. For the upstream second filter assembly 5, the level of the inlet 501 is lower than that of the outlet 502 thereof. For the downstream second filter assembly 5, the level of the inlet 501 of is higher than that of the outlet 502 thereof. The aforesaid arrangement of the second filter assemblies 5 and the arrangement of the discharge unit 74 are designed to enhance gas-discharging effect, and to reduce the accumulation of gas in the receiving spaces 50 of the second filter assemblies 5.

In addition, the flow meter 76 is connected to the transport pipe 72 that connects the outlet 202 of the water-generating device 2 and the inlet 401 of the first filter assembly 4, and is disposed upstream of the upstream measuring unit 75. It should be noted that, when the discharge unit 74 is not turned on in time to discharge the accumulated gas, or either one or both of the first filter assembly 4 and the second filter assembly 5 is clogged, the water flow speed will be reduced (e.g., drop to 0.4 L/min from 0.9∼2 L/min) and will be detected by the flow meter 76. The reduced water flow speed might seriously affect water generating speed and water quality (such as increased bacteria number, changes in taste, etc.) of the water composition 9. Thus, when the reduced water flow speed is detected by the flow meter 76, the discharge unit 74 can be turned on in time to discharge the gas accumulating in the transport pipe 72. Moreover, by incorporating the information about ΔV, reduction in water flow speed, and period of use of the first filter assembly 4 and the second filter assembly 5, the need for replacing the first filter assembly 4 and the second filter assembly 5 can be determined by the user.

The disclosure will be further described by way of the following examples. However, it should be understood that the following examples are solely intended for the purpose of illustration and should not be construed as limiting the disclosure in practice.

### EXAMPLES:

### Preparation 1: Water composition of Example 1 and method for making the same

A water composition of Example 1 is the first embodiment of the water composition 9 as mentioned above, and the method for making the water composition of Example 1 is based on the method described in FIGS. 2 to 5.

First, a small amount of drinking water was filled in a first container having a capacity of 1 L. Next, a bottom opening of a second container having a capacity of 30 mL was covered with a hydrophilic membrane filter having an average pore diameter of 220 nm, and the second container was filled with 150 mg of nano non-porous silicon (purity: 99.35%, average diameter: 200 nm). After filling the second container with drinking water, a top opening of the second container was covered with another hydrophilic membrane filter having an average pore diameter of 220 nm. Then, the top and bottom openings of the second container were respectively covered with two covers each having a through hole. The second container filled with the nano non-porous silicon and the drinking water was placed in the first container. The nano non-porous silicon was obtained by grinding a silicon wafer having a purity of 9N. Afterwards, a remaining space in the first container was filled with the drinking water, and then the first container was sealed. Finally, the first container was inverted in an upside down position. The drinking water was continuously reacted with the nano non-porous silicon to generate silicic acid and hydrogen gas so as to obtain the water composition of Example 1. Two samples of the water composition 9 were prepared. One of the samples of the water composition 9 was subjected to ORP and the silicic acid concentration measurements after 13 days, and the other one of the samples of the water composition 9 was subjected to such measurements after 28 days.

The ORP of the water composition of Example 1 was measured using electrodes (Manufacturer: JAQUA; Model: EO221) and an oxidation-reduction potential (ORP) analyzer (Manufacturer: Horiba Ltd.; Model: F-51) . The water composition was subjected to colorimetric measurement using a silicate test kit (MColortest™, Merck) to determine silicic acid concentration. The analyzed data of the water composition of Example 1 is shown in Table 1.

**Table 1**

| Analyzed data | Drinking water | Water composition | |
|---|---|---|---|
| | | After 13 days | After 28 days |
| ORP (mV) | 212 | -203 | -446 |
| Silicic acid concentration (mg/L) | 0 | 1.5 | 3 |

When the first container was opened after 28 days for determining the concentration of silicic acid and the ORP value, the concentration of the silicic acid and ORP value of the water composition of Example 1 were 3 mg/L and -446 mV, respectively. However, the applicants found that when the silicic acid and hydrogen gas in the second container 82 diffused evenly throughout the first container 81, the silicic acid present in the water composition is in a concentration of not lower than 11 mg/mL, and the ORP value might be lower than -500 mV.

### Preparation 2: Water composition of Example 2 and method for making the same

A water composition 9 of Example 2 was made using the second embodiment of a filter assembly 4 (i.e., the filter assembly 4 shown in FIG. 11, in which a binder 423 is used to bind a portion of the silicon material (e.g., nano silicon) 422 and the activated carbon 4211.

Referring again to FIG. 11, drinking water was introduced into the inlet 401 of the filter assembly 4, and was reacted with the silicon material 422 of the filter 42 to generate silicic acid and hydrogen gas 92 so as to obtain the water composition 9 containing the silicic acid and the hydrogen gas 92. The water composition 9 of Example 2 then flowed out of the filter assembly 4 from the outlet 402. The specification and source of the silicon material 422 were the same as those used in Preparation 1. The activated carbon 4211 was a coconut shell activated carbon purchased from Goldstar Carbon Tech Inc., Taiwan, having a diameter between 150 and 380 µm, and the binder 423 was an ultra-high-molecular-weight polyethylene purchased from Celanese Corporation (Model No: GUR2122).

The silicic acid concentration and the ORP value of the water composition 9 of Example 2 were determined using the same method as used in Preparation 1, and the analyzed data are summarized in Table 2.

**Table 2**

| | ORP (mV) | Silicic acid concentration (mg/L) |
|---|---|---|
| Drinking water to be fed into the filter assembly | 280∼300 | 0.3 |
| Water composition obtained using the filter assembly (237 L) | -626 | 30 |

### Preparation 3: Filter and method for making the same

A filter as shown in FIG. 6 (i.e., the first embodiment of the filter 42) was made using the method for making the filter shown in FIG. 7. The specification and source of nano non-porous silicon used herein were the same as those used in Preparation 1. The activated carbon was purchased from Haycarb PLC (Model No: RWAP 1074), and had a diameter approximately between 0.425 mm and 1.7 mm (hereinafter referred to as HB activated carbon) .

First, 80 g of the nano non-porous silicon and alcohol with 99.5% purity were evenly mixed to obtain a nano non-porous silicon slurry with a solid content of 20%. Next, 270 g of the activated carbon was added to the nano non-porous silicon slurry and the obtained mixture was stirred evenly in order for the nano non-porous silicon in the nano non-porous silicon slurry to be deposited on the activated carbon (some of the nano non-porous silicon was situated in the micropores 4210) . Finally, the mixture was dried to remove the alcohol therein, thereby obtaining the filter. The weight percentage of the activated carbon and the nano non-porous silicon in the filter of Preparation 3 were 77.1 wt% and 22.9 wt%, respectively.

### Preparation 4: Filter assembly and method for making the same

A filter assembly as shown in FIG. 10 (i.e., the first embodiment of the filter assembly 4) was prepared. The specification and source of the nano non-porous silicon used herein were the same as those used in Preparation 1, and the activated carbon was the HB activated carbon as mentioned above.

First, 50 g of the activated carbon (bottom portion), 100 g of the nano non-porous silicon (middle portion), and 230 g of the activated carbon (top portion) were sequentially filled into a receiving space of a housing through an outlet of the housing. The weight percentage of the total activated carbon and the nano non-porous silicon were 73.7 wt% and 26.3 wt%, respectively. Then, the inlet and the outlet of the housing were respectively connected to a transport pipe of pipeline unit. A drinking water was then introduced into the receiving space from the inlet, and flowed toward the outlet. The nano non-porous silicon was moved by the water flow and then was deposited in the micropores 4210 of the activated carbon 4211. When the drinking water was reacted with the nano non-porous silicon, silicic acid and hydrogen gas were generated. The nano non-porous silicon deposited in the micropores of the activated carbon facilitates an increase in the amount of dissolved hydrogen as mentioned above.

### Preparation 5: Water composition of Example 3 and method for making the same

A water composition of Example 3 was prepared using the first embodiment of the water purification system (containing two first filter assemblies, i.e., upstream first filter assembly and downstream first filter assembly) and one second filter assembly. The two first filter assemblies were made using the method described in Preparation 4. The specification and source of nano non-porous silicon used herein were the same as those used in Preparation 1, and activated carbon was the HB activated carbon as mentioned above.

First, a drinking water produced by an R.O. device (Manufacturer: ADD; Model No. : 400P) was introduced from an outlet of the R. O. device into a membrane degasifier through a pipe to remove CO₂, O₂ and N₂ from the drinking water. The drinking water was then introduced from an outlet of the membrane degasifier into the upstream first filter assembly through a pipeline unit. The drinking water was then reacted with the nano non-porous silicon of the upstream first filter assembly to generate silicic acid and hydrogen gas so as to obtain the water composition. The water composition (containing the drinking water, silicic acid, and hydrogen gas) and a portion of the nano non-porous silicon were then introduced from an outlet of the upstream first filter assembly through a transport pipe of the pipeline unit to the downstream first filter assembly. The drinking water of the water composition was continuously reacted with the nano non-porous silicon filled in the downstream first filter assembly to generate silicic acid and hydrogen gas, thereby increasing the amount of the silicic acid and the dissolved hydrogen.

Subsequently, the water composition was delivered from an outlet of the downstream first filter assembly to the second filter assembly through the pipeline unit. The second filter assembly contained a plurality of hollow fiber membranes (Manufacturer: Sampo Corporation; Model: FJ-V1203BL). All or a part of the nano non-porous silicon was adsorbed onto the hollow fiber membranes. The water composition was then introduced from an outlet of the second filter assembly through the pipeline unit to an ultraviolet light sterilization unit (Manufacturer: KC-FLOW; Model: 16W-2GPM) to disinfect the water composition.

Finally, the disinfected water composition (i.e., water composition of Example 3) then flowed out of the water purification system from an outlet of the ultraviolet light sterilization unit.

It should be noted that the filter used in the first filter assemblies of the water purification system may be other filter as mentioned above.

The drinking water to be fed into the first filter assemblies, the water composition immediately flowing from the outlet of the ultraviolet light sterilization unit, and the total water composition produced using the water purification system and collected for 15 days, were subjected to the ORP value and the silicic acid concentration measurements. The data are summarized in Table 3.

**Table 3**

| | ORP (mV) | Silicic acid concentration (mg/L) |
|---|---|---|
| Drinking water to be fed into the first filter assemblies | 280∼300 | 0.3 |
| Water composition flowing from the outlet of the ultraviolet light sterilization unit | <-500 | >42 |
| Total water composition collected for 15 days | -576 | >42 |

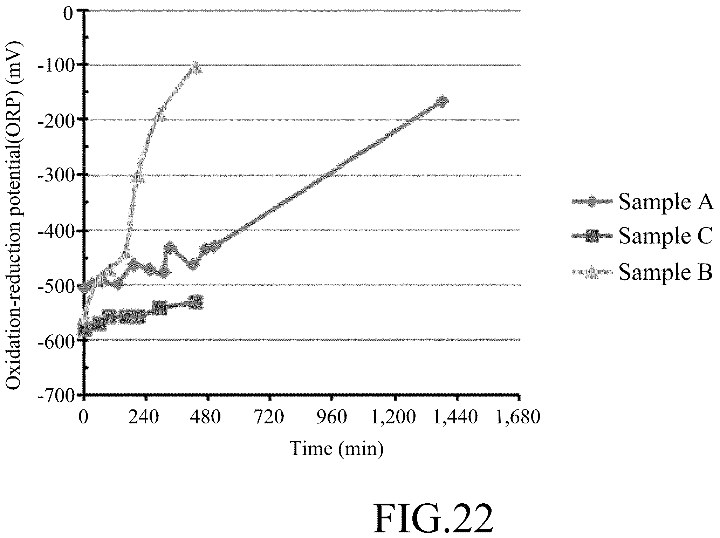

Furthermore, 900 mL of the water composition of Example 3 flowing from the outlet of the ultraviolet light sterilization unit was directly received and sealed in a 1 L bottle to obtain Sample A with a filling rate of 90%. 230 mL of the water composition of Example 3 flowing from the outlet of the ultraviolet light sterilization unit was directly received and sealed in a 1 L bottle to obtain Sample B with a filling rate of 23%. 215 mL of the water composition of Example 3 flowing from the outlet of the ultraviolet light sterilization unit was directly received and sealed in a 230 mL bottle to obtain Sample C with a filling rate of 93.48%. After the bottles of Samples A to C were opened, variation in ORP values of Samples A to C over time was recorded. Referring to FIG. 22, for Sample A, the ORP value at 60-minute mark (60 minutes after the bottles of Sample A were opened) is about -500 mV, and the OPR value at 432-minute mark had increased to -100 mV. For Sample B, the ORP value at 432-minute mark is below -450 mV. For Sample C, the ORP value at 432-minute mark is approximately -530 mV. Thus, it is revealed that the water composition of Example 3 of this disclosure has good stability of OPR.

### Application Example

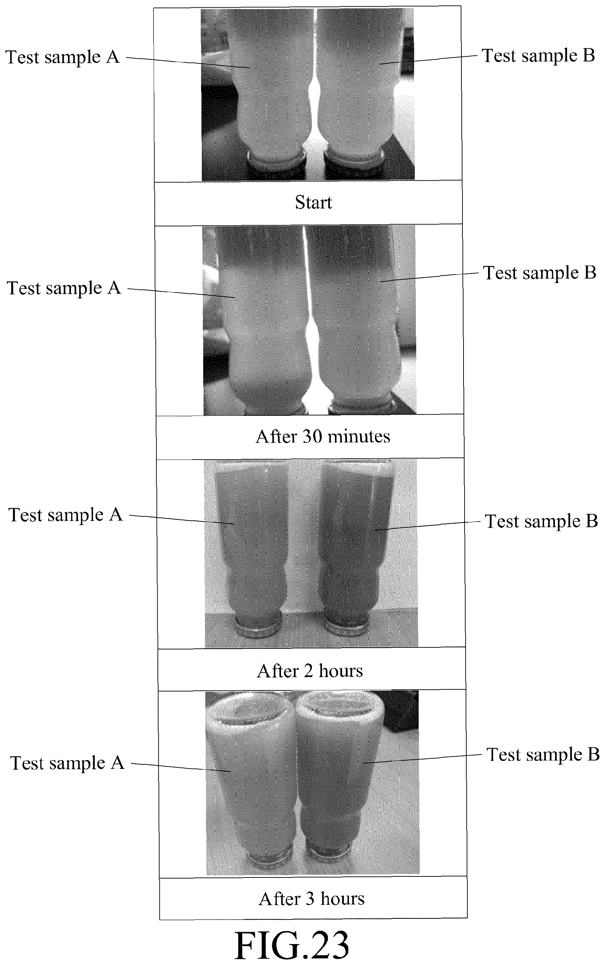

In order to further prove that the water composition of this disclosure has oxidation-reducing effect, apple juice was used in the Application Example. To be specific, 100 mL of apple juice was added into a 0.2 L glass bottle, and then 100 mL of the water composition of Example 3 was added into the bottle to obtain test sample A. To prepare test sample B (as a Comparative Example), 100 mL of apple juice was added into a 0.2 L glass bottle, and then 100 mL of the drinking water used in Example 3 (i.e., the drinking water to be fed into the filter assembly 4) was added into the bottle to obtain test sample B. Test samples A and B were sealed and then inverted upside down, and the color change of test samples A and B was observed. As shown by FIG. 23, the left glass bottle in each picture is test sample A and the right glass bottle in each picture is test sample B. Result showed that at 3 hours after the glass bottles were sealed, test sample B (i.e., right glass bottle) has changed from grey color to dark grey color, which indicates that the apple juice of test sample B was significantly oxidized. Moreover, the color of test sample B was darker than that of test sample A after 2 hours and also after 3 hours, which indicates that the water composition of Example 3 was able to reduce oxidation rate.

In summary, the filter, filter assembly, and the water purification system (which contains the silicon material or the combination of the silicon material and the carrier) of this disclosure can be used to produce the water composition containing the silicic acid and the hydrogen gas by reacting water with the silicon material. The obtained water composition containing silicic acid is likely to be beneficial for the formation of human connective tissues, and also exhibits oxidation-reducing effect. The water composition 9 of this disclosure is relatively safe compared to the conventional hydrogen-dissolved drinking water that contains magnesium hydroxide.

In the description above, for the purposes of explanation, numerous specific details have been set forth in order to provide a thorough understanding of the embodiments. It will be apparent, however, to one skilled in the art, that one or more other embodiments may be practiced without some of these specific details . It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. A filter (42) for producing a water composition (9) containing silicic acid and hydrogen gas (92); **characterized by** a carrier (421) and a nano silicon material (422) supported on said carrier (421), said nano silicon material (422) is present in an amount not lower than 10 wt% based on the weight of said filter (42).

2. The filter (42) as claimed in claim 1, **characterized in that** said carrier (421) is selected from the group consisting of activated carbon, hollow fiber membrane (HFM), bamboo charcoal, porphyritic andesite, quartz sand, fiber, ceramics and combinations thereof, and said nano silicon material (422) has an average diameter ranging from 50 nm to 300 nm and is selected from the group consisting of non-porous silicon, porous silicon, granulated silicon, silicon nanowire, and combinations thereof.

3. The filter (42) as claimed in claim 1 or 2, **characterized in that** said carrier (421) is activated carbon (4211) having a plurality of micropores (4210), said nano silicon material (422) being deposited in micropores (4210) of said activated carbon (4211).

4. The filter (42) as claimed in claim 3, further **characterized by** a binder (423) to bind said nano silicon material (422) to said activated carbon (4211), said binder (423) and said activated carbon (4211) cooperatively form a sintered activated carbon.

5. The filter (42) as claimed in claim 1 or 2, **characterized in that** said carrier (421) is hollow fiber membrane (4212) having a hollow fiber (4213) formed with a plurality of through holes (4210) formed thereon.

6. A filter assembly (4) for producing a water composition (9) **characterized by**:
a housing (41) defining a receiving space (40) and including an inlet (401) and an outlet (402), said inlet and said outlet (401,402) being fluidly communicated with said receiving space (40); and
a filter (42) as claimed in any one of claims 1 to 5, which is received in said receiving space (40) of said housing (41).

7. The filter assembly (4) as claimed in claim 6, **characterized in that** said carrier (421) is a combination of said activated carbon (4211) and said hollow fiber membrane (4212), said activated carbon (4211) being disposed proximate to said inlet (401), and said hollow fiber membrane (4212) being disposed proximate to said outlet (402).

8. The filter assembly (4) as claimed in claim 7, **characterized in that** one of said activated carbon (4211) and said hollow fiber membrane (4212) is disposed to surround the other of said activated carbon (4211) and said hollow fiber membrane (4212).

9. A water purification system for producing a water composition (9), **characterized by** at least one filter assembly (4) as claimed in any one of claims 6 to 8 as a first filter assembly (4).

10. The water purification system as claimed in claim 9, further **characterized by**:
at least one second filter assembly (5) that includes a housing (51) defining a receiving space (50) and a porous material (52) received in said receiving space (50), said housing (51) including an inlet (501) and an outlet (502) fluidly communicating with said receiving space (50); and
a pipeline unit (7) having at least one transport pipe (72) fluidly connecting said first and second filter assemblies (4,5).

11. The water purification system as claimed in claim 10, **characterized in that** said porous material (52) of said at least one second filter assembly (5) is selected from the group consisting of activated carbon (521), hollow fiber membrane (522) and the combination thereof.

12. The water purification system as claimed in claim 10 or 11, **characterized in that** said water purification system includes a plurality of said second filter assemblies (5), and said porous material (52) of each second filter assembly (5) is independently selected from the group consisting of activated carbon (521), hollow fiber membrane (522) and the combination thereof.

13. The water purification system as claimed in any one of claims 10 to 12, further **characterized by** a discharge unit (74) disposed downstream of said first filter assembly (4) and operationally connected to said pipeline unit (7) to discharge a liquid, a gas or the combination thereof from said pipeline unit (7).

14. The water purification system as claimed in any one of claims 9 to 13, further **characterized by** a degassing unit (3) disposed upstream of said first filter assembly (4) to remove gas from a to-be-treated water (91').

15. The water purification system as claimed in any one of claims 9 to 14, further **characterized by** two measuring units (75) respectively disposed upstream and downstream of said first filter assembly (4) to respectively determine total dissolved solids of a to-be-treated water (91') and a water composition (9) produced by the water purification system.

## Patentansprüche

1. Filter (42) zum Erzeugen einer Wasserzusammensetzung (9), die Kieselsäure und Wasserstoffgas (92) enthält; **gekennzeichnet durch** einen Träger (421) und ein Nanosilicium-Material (422), das auf dem Träger (421) gelagert ist, das Nanosilicium-Material (422) in einer Menge von nicht weniger als 10 Gew.-% basierend auf dem Gewicht des Filters (42) vorhanden ist.

2. Filter (42) nach Anspruch 1, **dadurch gekennzeichnet, dass** der Träger (421) aus der Gruppe ausgewählt ist, die aus Aktivkohle, Hohlfasermembran (HFM), Bambuskohle, porphyrisches Andesit, Quarzsand, Faser, Keramik und Kombinationen daraus besteht, und das Nanosilicium-Material (422) einen durchschnittlichen Durchmesser im Bereich von 50 nm bis 300 nm aufweist und aus der Gruppe ausgewählt ist, die aus nicht-porösem Silicium, porösem Silicium, granuliertem Silicium, Silicium-Nanodraht und Kombinationen daraus besteht.

3. Filter (42) nach Anspruch 1 oder 2, **dadurch gekennzeichnet, dass** der Träger (421) Aktivkohle (4211) ist, die eine Vielzahl von Mikroporen (4210) aufweist, wobei das Nanosilicium-Material (422) in Mikroporen (4210) der Aktivkohle (4211) abgelagert ist.

4. Filter (42) nach Anspruch 3, weiter **gekennzeichnet durch** einen Binder (423) zum Binden des Nanosilicium-Materials (422) an die Aktivkohle (4211), wobei der Binder (423) und die Aktivkohle (4211) zusammenwirkend eine gesinterte Aktivkohle bilden.

5. Filter (42) nach Anspruch 1 oder 2, **dadurch gekennzeichnet, dass** der Träger (421) eine Hohlfasermembran (4212) ist, die eine Hohlfaser (4213) aufweist, die mit einer Vielzahl von darauf gebildeten Durchgangslöchern (4210) gebildet ist.

6. Filteranordnung (4) zum Erzeugen einer Wasserzusammensetzung (9), **gekennzeichnet durch**:
ein Gehäuse (41), das einen Aufnahmeraum (40) definiert und einen Einlass (401) und einen Auslass (402) einschließt, wobei der Einlass und der Auslass (401, 402) mit dem Aufnahmeraum (40) fluidtechnisch kommuniziert sind; und
einen Filter (42) nach einem der Ansprüche 1 bis 5, der in dem Aufnahmeraum (40) des Gehäuses (41) aufgenommen ist.

7. Filteranordnung (4) nach Anspruch 6, **dadurch gekennzeichnet, dass** der Träger (421) eine Kombination von der Aktivkohle (4211) und der Hohlfasermembran (4212) ist, wobei die Aktivkohle (4211) nahe dem Einlass (401) angeordnet ist, und die Hohlfasermembran (4212) nahe dem Auslass (402) angeordnet ist.

8. Filteranordnung (4) nach Anspruch 7, **dadurch gekennzeichnet, dass** eine von der Aktivkohle (4211) und der Hohlfasermembran (4212) angeordnet ist, um die andere von der Aktivkohle (4211) und der Hohlfasermembran (4212) zu umgeben.

9. Wasserreinigungssystem zum Erzeugen einer Wasserzusammensetzung (9), **gekennzeichnet durch** mindestens eine Filteranordnung (4) nach einem der Ansprüche 6 bis 8 als erste Filteranordnung (4).

10. Wasserreinigungssystem nach Anspruch 9, weiter **gekennzeichnet durch**:
mindestens eine zweite Filteranordnung (5), die ein Gehäuse (51) einschließt, das einen Aufnahmeraum (50) definiert und ein poröses Material (52), das in dem Aufnahmeraum (50) aufgenommen ist, wobei das Gehäuse (51) einen Einlass (501) und einen Auslass (502) einschließt, die mit dem Aufnahmeraum (50) fluidtechnisch kommunizieren; und
eine Rohrleitungseinheit (7), die mindestens ein Transportrohr (72) aufweist, das die erste und zweite Filteranordnung (4, 5) fluidtechnisch verbindet.

11. Wasserreinigungssystem nach Anspruch 10, **dadurch gekennzeichnet, dass** das poröse Material (52) der mindestens einen zweiten Filteranordnung (5) aus der Gruppe ausgewählt ist, die aus Aktivkohle (521), Hohlfasermembran (522) und der Kombination daraus besteht.

12. Wasserreinigungssystem nach Anspruch 10 oder 11, **dadurch gekennzeichnet, dass** das Wasserreinigungssystem eine Vielzahl der zweiten Filteranordnungen (5) einschließt, und das poröse Material (52) von jeder zweiten Filteranordnung (5) unabhängig aus der Gruppe ausgewählt ist, die aus Aktivkohle (521), Hohlfasermembran (522) und der Kombination daraus besteht.

13. Wasserreinigungssystem nach einem der Ansprüche 10 bis 12, weiter **gekennzeichnet durch** eine Austrageeinheit (74), die stromabwärts der ersten Filteranordnung (4) angeordnet ist, und betrieblich mit der Rohrleitungseinheit (7) verbunden ist, um eine Flüssigkeit, ein Gas oder eine Kombination daraus aus der Rohrleitungseinheit (7) auszutragen.

14. Wasserreinigungssystem nach einem der Ansprüche 9 bis 13, weiter **gekennzeichnet durch** eine Entgasungseinheit (3), die stromaufwärts der ersten Filteranordnung (4) angeordnet ist, um Gas aus einem zu behandelnden Wasser (91') zu entfernen.

15. Wasserreinigungssystem nach einem der Ansprüche 9 bis 14, weiter **gekennzeichnet durch** zwei Messeinheiten (75), die jeweils stromaufwärts und stromabwärts der ersten Filteranordnung (4) angeordnet sind, um jeweils Filtrattrockenrückstände eines zu behandelnden Wassers (91') und einer Wasserzusammensetzung (9) zu bestimmen, die von dem Wasserreinigungssystem erzeugt werden.

## Revendications

1. Filtre (42) pour produire une composition d'eau (9) contenant de l'acide silicique et de l'hydrogène gazeux (92) ;
**caractérisé par** un support (421) et un matériau de nano-silicium (422) supporté sur ledit support (421), ledit matériau de nano-silicium (422) est présent en une quantité non inférieure à 10% en poids par rapport au poids dudit filtre (42).

2. Filtre (42) tel que revendiqué dans la revendication 1, **caractérisé en ce que** ledit support (421) est choisi dans le groupe constitué de charbon actif, de membrane à fibres creuses (HFM), de charbon de bois de bambou, d'andésite porphyrique, de sable de quartz, de fibre, de céramique et de combinaisons de ceux-ci, et ledit matériau de nano-silicium (422) a un diamètre moyen compris entre 50 nm et 300 nm et est choisi dans le groupe constitué de silicium non poreux, de silicium poreux, de silicium granulé, de nanofil de silicium et de combinaisons de ceux-ci.

3. Filtre (42) tel que revendiqué dans la revendication 1 ou 2, **caractérisé en ce que** ledit support (421) est du charbon actif (4211) ayant une pluralité de micropores (4210), ledit matériau de nano-silicium (422) étant déposé dans des micropores (4210) dudit charbon actif (4211).

4. Filtre (42) tel que revendiqué dans la revendication 3, **caractérisé en outre par** un liant (423) pour lier ledit matériau de nano-silicium (422) audit charbon actif (4211), ledit liant (423) et ledit charbon actif (4211) forment coopérativement un charbon actif fritté.

5. Filtre (42) tel que revendiqué dans la revendication 1 ou 2, **caractérisé en ce que** ledit support (421) est une membrane à fibres creuses (4212) ayant une fibre creuse (4213) formée avec une pluralité de trous traversants (4210) formés dessus.

6. Ensemble filtre (4) pour produire une composition d'eau (9) **caractérisé par** :
un boîtier (41) définissant un espace de réception (40) et comportant une entrée (401) et une sortie (402), ladite entrée et ladite sortie (401, 402) étant en communication fluidique avec ledit espace de réception (40) ; et
un filtre (42) tel que revendiqué dans l'une quelconque des revendications 1 à 5, qui est reçu dans ledit espace de réception (40) dudit boîtier (41).

7. Ensemble filtre (4) tel que revendiqué dans la revendication 6, **caractérisé en ce que** ledit support (421) est une combinaison dudit charbon actif (4211) et de ladite membrane à fibres creuses (4212), ledit charbon actif (4211) étant disposé à proximité de ladite entrée (401) et ladite membrane à fibres creuses (4212) étant disposée à proximité de ladite sortie (402).

8. Ensemble filtre (4) tel que revendiqué dans la revendication 7, **caractérisé en ce que** l'un(e) dudit charbon actif (4211) et de ladite membrane à fibres creuses (4212) est disposé(e) de manière à entourer l'autre dudit charbon actif (4211) et de ladite membrane à fibres creuses (4212).

9. Système de purification d'eau pour produire une composition d'eau (9), **caractérisé par** au moins un ensemble filtre (4) tel que revendiqué dans l'une quelconque des revendications 6 à 8 en tant que premier ensemble filtre (4).

10. Système de purification d'eau tel que revendiqué dans la revendication 9, **caractérisé en outre par** :
au moins un deuxième ensemble filtre (5) qui comporte un boîtier (51) définissant un espace de réception (50) et un matériau poreux (52) reçu dans ledit espace de réception (50), ledit boîtier (51) comportant une entrée (501) et une sortie (502) en communication fluidique avec ledit espace de réception (50) ; et
une unité de conduit (7) ayant au moins un tuyau de transport (72) raccordant de manière fluidique lesdits premier et deuxième ensembles filtre (4,5).

11. Système de purification d'eau tel que revendiqué dans la revendication 10, **caractérisé en ce que** ledit matériau poreux (52) dudit au moins un deuxième ensemble filtre (5) est choisi dans le groupe constitué du charbon actif (521), de la membrane à fibres creuses (522) et de la combinaison de ceux-ci.

12. Système de purification d'eau tel que revendiqué dans la revendication 10 ou 11, **caractérisé en ce que** ledit système de purification d'eau comporte une pluralité desdits deuxièmes ensembles filtre (5), et ledit matériau poreux (52) de chaque deuxième ensemble filtre (5) est indépendamment choisi dans le groupe constitué du charbon actif (521) et de la membrane à fibres creuses (522) et de la combinaison de ceux-ci.

13. Système de purification d'eau tel que revendiqué dans l'une quelconque des revendications 10 à 12, **caractérisé en outre par** une unité d'évacuation (74) disposée en aval dudit premier ensemble filtre (4) et raccordée de manière fonctionnelle à ladite unité de conduit (7) pour évacuer un liquide, un gaz ou une combinaison de ceux-ci provenant de ladite unité de conduit (7).

14. Système de purification d'eau tel que revendiqué dans l'une quelconque des revendications 9 à 13, **caractérisé en outre par** une unité de dégazage (3) disposée en amont dudit premier ensemble filtre (4) pour éliminer le gaz d'une eau à traiter (91').

15. Système de purification d'eau tel que revendiqué dans l'une quelconque des revendications 9 à 14, **caractérisé en outre par** deux unités de mesure (75) disposées respectivement en amont et en aval dudit premier ensemble filtre (4) pour déterminer respectivement les matières solides totales dissoutes d'une eau à traiter (91') et d'une composition d'eau (9) produite par le système de purification d'eau.
